# EUROPÄISCHE PATENTSCHRIFT

(11) **EP 3 593 780 B1**
(45) Veröffentlichungstag und Bekanntmachung des Hinweises auf die Patenterteilung: **11.06.2025**
(21) Anmeldenummer: 19178886.8
(22) Anmeldetag: 19.10.2015
(51) Int. Cl.: A61G 12/00, F16M 13/02

(54) **MONTAGEVORRICHTUNG MIT DREHKUPPLUNG FÜR EINE STATIVVORRICHTUNG**
MOUNTING DEVICE WITH ROTARY COUPLING FOR A STAND DEVICE
DISPOSITIF DE MONTAGE À ACCOUPLEMENT ROTATIF POUR UN DISPOSITIF DE TRÉPIED

(30) Priorität: 17.10.2014 EP 14003556
(43) Veröffentlichungstag der Anmeldung: 15.01.2020
(62) Teilanmeldung aus: 15781872.5
(73) Patentinhaber: Ondal Medical Systems GmbH, 36088 Hünfeld (DE)
(72) Erfinder: Oginski, Stefan, 36041 Fulda (DE); Höser, Markus, 36142 Tann (DE); Göbel, Andreas, 36132 Eiterfeld (DE)
(74) Vertreter: Graf von Stosch Patentanwaltsgesellschaft mbH

(56) Entgegenhaltungen:
- DE-A1- 102012 001 197
- DE-A1- 3 100 819
- US-A- 4 673 154
- US-A- 4 738 369

## Beschreibung

Die vorliegende Erfindung betrifft eine Montagevorrichtung für eine Stativvorrichtung zur Anordnung im Operationssaal und zum örtlichen Positionieren oder Verlagern einer medizintechnischen Einrichtung im Operationssaal, umfassend: eine sich in einer Längsrichtung entlang einer Drehachse erstreckende Montageeinrichtung mit einer in Längsrichtung ausgerichteten Kavität zur Aufnahme eines drehbar lagerbaren Verbindungsbauteils der Stativvorrichtung; und eine Justageeinrichtung zur Anordnung des Verbindungsbauteils in einer vordefinierbaren Position relativ zur Montageeinrichtung. Die vorliegende Erfindung betrifft insbesondere eine Montagevorrichtung mit den Merkmalen des Anspruchs 1 und ein Montagssystem mit den Merkmalen des Anspruchs 13.

Stative, insbesondere Deckenstative wie z.B. Deckenversorgungseinheiten, Monitorträger, oder so genannte Federarme oder Zentralachsen, weisen meist einen oder mehrere in Bezug auf eine Vertikalposition starr angeordneten oder höhenverstellbaren Träger auf, mittels welchen eine daran befestigte medizintechnische Einrichtung bewegt und positioniert werden kann, z.B. im Operationssaal, insbesondere auch auf einer Intensivstation. An den Stativen sind häufig Versorgungseinheiten montiert, an welchen z.B. medizinisch-elektrische Endgeräte angeordnet sind, die z.B. während einer Operation mit den benötigten Medien versorgt werden. Die Träger definieren dabei einen Aktionsradius der medizintechnischen Einrichtung, in welchem die medizintechnische Einrichtung positioniert werden kann. Die Träger können meist zumindest um mindestens eine drehbare Verbindung, insbesondere ein Drehgelenk verdreht werden. Wahlweise sind die Träger auch höhenverstellbar und/oder um eine zumindest annähernd horizontal ausgerichtete Achse in der Höhe verschwenkbar angeordnet.

Die Montage des Stativs erfolgt häufig an der Raumdecke bzw. angrenzend an eine Zwischendecke des Operationssaals. Die Zwischendecke selbst dient dabei zum Lagern von z.B. Kabeln, nicht aber zum Abstützen des Stativs. Hierzu weist das Stativ z.B. ein Deckenrohr auf. Dabei muss häufig eine Anpassung der Drehposition des Stativs relativ zur Raumdecke oder zu einem Deckenflansch erfolgen. Über die Drehposition des Stativs relativ zu einem Montagepunkt an der Raumdecke kann der Aktionsradius vorgegeben werden. Zur Montage des Stativs können z.B. Montagevorrichtungen mit auf der Spindel aufgeschrumpften Scheiben oder Flanschplatten verwendet werden. Die aufgeschrumpften Scheiben können radial mit dem Deckenrohr verschraubt sein. Diese Lösung hat jedoch konstruktive Nachteile, insbesondere aufgrund spezieller Spaltmaße und Bauteiltoleranzen. Die Flanschplatten können jeweils mit der Spindel und dem Deckenrohr verbunden sein, ermöglichen in der vorbekannten Art jedoch nicht auf einfache Weise, die Drehposition der Spindel exakt einzustellen.

Die US 4,673,154 A zeigt ein Gerät, an dem Gegenstände aufgehängt werden können, bestehend aus einem Aufhängungspfosten, einem ersten Rotor, einem ersten Lager mit einer Welle und einem um die Welle drehbaren Lagerring. Das erste Ende der Welle des ersten Lagers ist fest am Aufhängungspfosten befestigt. Der Rotor wird vom Lagerring des ersten Lagers getragen und dreht sich um die Achse der Welle des ersten Lagers. Ein erster Lagerkuppler ist fest mit der Welle des ersten Lagers verbunden und dafür ausgelegt, ein Ende der Welle eines zweiten Lagers, das dem ersten Lager ähnlich ist, in fester Verbindung aufzunehmen. Eine Halterungsarmbaugruppe für Gegenstände wird vom Rotor getragen.

Die DE 10 2012 001 197 A1 zeigt ein Stativ für einen Infusionsständer oder als Halterung für medizinische Geräte umfassend wenigstens ein äußeres Rohr und ein inneres Rohr, wobei das innere Rohr in dem äußeren Rohr einschiebbar ist und die Rohre in deren Längsrichtung relativ zueinander bewegbar und in wenigstens einer Längsrichtung arretierbar sind, und wenigstens ein selbsttätiges Betätigungselement zum zumindest zeitweisen Lösen der Arretierungen zwischen den Rohren, welches sich dadurch auszeichnet, dass das innere Rohr eine Arretiervorrichtung aufweist, welche im arretierten Zustand eine Drehbewegung des inneren Rohrs relativ zu dem äußeren Rohr vermeidet.

Die DE 31 00 819 A1 zeigt ein Deckendrehlager für die Halterung von in ihrer Lage durch Bremsen und Anschläge feststellbaren medizinischen Geräten. Es enthält die Anschlüsse für die Energie- und Gasversorgungsleitungen.

Die US 4,738,369 A zeigt eine Halterung für Patientenüberwachungsgeräte, bereitgestellt durch eine vertikale Säule, die von der Decke mittels einer Laufwagenanordnung aufgehängt ist, die für eine seitliche Bewegung auf Rollenanordnungen entlang eines Paares paralleler, deckenmontierter Schienen montiert ist. Die Säule wird durch die Rollenanordnungen stabilisiert, die sowohl Rollen umfassen, die auf den Schienenflanschen laufen, als auch Rollen, die unterhalb der Schienen angeordnet sind und ein Wanken der Säule verhindern sollen.

Es ist eine Aufgabe der vorliegenden Erfindung, eine Montagevorrichtung bereitzustellen, mittels welcher eine Ausrichtung der Spindel und damit eine Festlegung des Aktionsradius der Stativvorrichtung im Operationssaal auf möglichst exakte Weise erfolgen und eingestellt werden kann. Die Aufgabe besteht insbesondere auch darin, eine Montagevorrichtung bereitzustellen, mittels welcher ein Einstellen oder Nachjustieren des Aktionsradius auf besonders einfache Weise erfolgen kann. Die Montagevorrichtung soll sich dabei bevorzugt auch durch eine hohe Tragfähigkeit auszeichnen und zur Aufnahme und Weiterleitung von hohen Gewichtskräften geeignet sein. Bevorzugt weist die Montagevorrichtung dabei einen einfachen konstruktiven Aufbau auf und ist kostengünstig herstellbar. Bevorzugt ist die Montagevorrichtung dabei auch auf einfache Weise montierbar oder einstellbar.

Diese Aufgabe wird gelöst durch eine Montagevorrichtung für eine Stativvorrichtung zur Anordnung im Operationssaal und zum örtlichen Positionieren oder Verlagern einer medizintechnischen Einrichtung im Operationssaal, insbesondere durch eine Drehbewegung, mit den Merkmalen des Anspruchs 1. Vorteilhafte Ausführungen ergeben sich aus den Unteransprüchen.

Entsprechend wird eine Montagevorrichtung für eine Stativvorrichtung zur Anordnung im Operationssaal und zum örtlichen Positionieren oder Verlagern einer medizintechnischen Einrichtung im Operationssaal, insbesondere durch eine Drehbewegung, vorgeschlagen, umfassend unter anderem: eine sich in einer Längsrichtung entlang einer Drehachse erstreckende Montageeinrichtung mit einer in Längsrichtung ausgerichteten, insbesondere zylindrischen Kavität zur Aufnahme eines drehbar lagerbaren Verbindungsbauteils der Stativvorrichtung, insbesondere einer Spindel; eine Justageeinrichtung zur verdrehfesten Anordnung des Verbindungsbauteils in einer vordefinierbaren Position relativ zur Montageeinrichtung, wobei die Justageeinrichtung drehfest mit dem Verbindungsteil verbindbar ausgebildet ist; und ein Verdrehsicherungselement; wobei die Montagevorrichtung eine um die Drehachse einstellbare Drehkupplung zum Lagern des Verbindungsbauteils an der Montageeinrichtung bildet. Hierdurch kann ein Einstellen oder Nachjustieren der relativen Drehposition der Spindel relativ zur Montagevorrichtung auf einfache Weise erfolgen. Der Aktionsradius des Stativs kann auf einfache Weise festgelegt werden.

Bevorzugt kann die Drehkupplung ohne Demontage irgendwelcher Komponenten der Montagevorrichtung oder Stativvorrichtung eingestellt werden, abgesehen von einer Verkleidung bzw. einzelnen abnehmbaren Verkleidungsteilen.

Bevorzugt ist die Montageeinrichtung eingerichtet, in einem ersten Zustand das Verbindungsbauteil verdrehbar und axialfest zu lagern, und in einem zweiten Zustand das Verbindungsbauteil verdrehfest und axialfest zu lagern, insbesondere in einem mit der Justageeinrichtung gekuppelten Zustand. Dies ermöglicht in Verbindung mit der Justageeinrichtung die Einstellbarkeit von definierten Verdrehwinkelpositionen.

Als Stativvorrichtung ist dabei bevorzugt eine Vorrichtung zum Halten, ortsfesten Anordnen/Positionieren und/oder Verlagern mindestens einer medizintechnische Einrichtung zu verstehen, die an einer Wand (in einem Wandlager) oder einer Raumdecke oder auch am Boden eines Operationssaals oder irgendeines anderen Raumes für medizinische Zwecke fest montiert oder positioniert werden kann, also z.B. ein Deckenstativ. Die Stativvorrichtung ist dann nicht vollkommen frei im Operationssaal verlagerbar, sondern kann nur in einem bestimmten Aktionsradius verlagert werden, insbesondere relativ zu einem an einer Raumdecke oder Wand des Operationssaals angeordneten Befestigungspunkt bzw. Montagepunkt. Die Stativvorrichtung kann als an einer Raumdecke montierte Deckenversorgungseinheit ausgebildet sein und eine oder mehrere Versorgungskonsolen aufweisen, welche an einem oder zwei Tragarmen gelagert und positionierbar ist. Die Stativvorrichtung kann auch als Monitorträger ausgebildet sein. Die Stativvorrichtung kann auch als so genannter, insbesondere an einer Wand montierter Federarm ausgebildet sein und z.B. eine Leuchte aufweisen. Die Stativvorrichtung kann auch als so genannte, insbesondere an einer Raumdecke montierte Zentralachse ausgebildet sein und eine Mehrzahl von Tragsystemen mit jeweils mindestens einem Träger aufweisen, an welchem z.B. ein Monitor oder eine Leuchte gelagert ist. Bevorzugt weist die Stativvorrichtung mindestens zwei Tragarme auf.

Als medizintechnische Einrichtung ist dabei bevorzugt eine Versorgungskonsole zu verstehen, mittels welcher Mittel für eine Versorgung eines Patienten und/oder Instrumente für einen Operateur und/oder Licht, Reinluft oder andere im Operationssaal benötigte Medien bereitgestellt werden können. Die medizintechnische Einrichtung weist bevorzugt irgendein Bedienpanel und/oder irgendeine Anzeigevorrichtung zum grafischen Darstellen von z.B. Patientendaten auf.

Als Montageeinrichtung ist dabei eine Einrichtung zu verstehen, mittels welcher die Stativvorrichtung an einer Raumdecke montierbar ist und welche eine Kupplung mit einem Verbindungsbauteil der Stativvorrichtung bildet, insbesondere eine Drehkupplung mit einer Spindel. Die Montageeinrichtung kann einen Deckenflansch umfassen oder mittels eines oder mehrerer Halter an einen Deckenflansch montiert werden.

Als Verbindungsbauteil ist dabei bevorzugt ein Bauteil zu verstehen, mittels welchem einzelne Träger der Stativvorrichtung mit der Montagevorrichtung und bevorzugt auch untereinander verbunden werden können. Bei einer Stativvorrichtung in Form einer so genannten Zentralachse kann das Verbindungsbauteil als zentral angeordnete Spindel ausgebildet sein, an welcher mehrere Träger oder Tragarme gelagert sind.

Als Justageeinrichtung ist dabei bevorzugt eine Einrichtung zu verstehen, mittels welcher eine bestimmte Relativposition des Verbindungsbauteils relativ zur Montageeinrichtung justiert bzw. eingestellt werden kann. Mittels der Justageeinrichtung können einzelne Relativpositionen vordefiniert werden. Die Justageeinrichtung ist bevorzugt verdrehfest am Verbindungsbauteil gelagert. Gemäß einer Variante übernimmt die Justageeinrichtung ausschließlich die Funktion einer Verdrehsicherung, nicht aber die Funktion einer axialen Sicherung.

Als Drehkupplung ist dabei bevorzugt eine Verbindung zu verstehen, mittels welcher eine Kupplung in einer bestimmten Drehposition sichergestellt werden kann, wobei eine relative Drehbewegung ermöglicht werden kann, sei es schrittweise, sei es stufenlos.

Als Operationssaal ist dabei auch ein Untersuchungsraum oder eine Intensivstation aufzufassen, also ein Raum zum Durchführen medizinischer Behandlungen oder Therapien.

Gemäß einem Ausführungsbeispiel ist die einstellbare Drehkupplung durch die Justageeinrichtung und die Montageeinrichtung gebildet, wobei die Justageeinrichtung in einer vorbestimmten Verdrehposition um die Drehachse relativ zur Montageeinrichtung positionierbar und verdrehfest lagerbar ist. Hierdurch kann ein Einstellen durch ein Verdrehen der Justageeinrichtung relativ zur Montageeinrichtung erfolgen. Die Justageeinrichtung ist eingerichtet, das Verbindungsbauteil verdrehfest an der Montageeinrichtung zu lagern.

Gemäß einer Variante ist die Justageeinrichtung separat vom Verbindungsbauteil und separat von der Montageeinrichtung als separate Komponente der Montagevorrichtung ausgebildet. Durch die separate Ausgestaltung der Justageeinrichtung von der Spindel kann eine Kupplung bereitgestellt werden, mittels welcher die Position der Spindel relativ zur Montageeinrichtung auf flexible und einfache Weise eingestellt werden kann. Bevorzugt ist die Justageeinrichtung formschlüssig mit dem Verbindungsbauteil kuppelbar, insbesondere in unterschiedlichen Axialpositionen. Diese Art Schnittstelle ermöglicht z.B. die Anordnung einer unterschiedlichen Anzahl von Trägern oder unterschiedlich dimensionierte Träger, ohne dass die Schnittstelle konstruktiv abgeändert werden muss.

Wahlweise kann die Justageeinrichtung auch zusammen mit dem Verbindungsbauteil eine einzige Komponente bilden. Beispielsweise kann die Justageeinrichtung stoffschlüssig mit dem Verbindungsbauteil verbunden sein, insbesondere verschweißt sein.

Die Kavität ist geometrisch korrespondierend zum Verbindungsbauteil ausgebildet und bildet ein Drehlager für das Verbindungsbauteil. Hierdurch kann das Verbindungsbauteil in der Montageeinrichtung zwecks Justage der Drehposition verdreht werden.

Bevorzugt sind die Montageeinrichtung und die Justiereinrichtung in axialer Richtung in Reihe zueinander angeordnet und überlappen jeweils das Verbindungsbauteil.

Gemäß einem Ausführungsbeispiel weist die Justageeinrichtung einen Verdrehanschlag auf, insbesondere eine Nut oder Feder, welcher geometrisch korrespondierend zu einem am Verbindungsbauteil angeordneten Verdrehanschlag, insbesondere Nut oder Feder, ausgebildet ist. Hierdurch kann eine Drehung der Justageeinrichtung eine Drehung der Spindel bewirken und umgekehrt. Das Einstellen der Drehposition der Justageeinrichtung relativ zur Montageeinrichtung ermöglicht somit unmittelbar auch ein Einstellen der Drehposition der Spindel im Operationssaal. Bevorzugt erstreckt sich die Nut zumindest annähernd in Längsrichtung. Der Verdrehanschlag kann wahlweise auch durch eine andere formschlüssige Kupplung sichergestellt werden, z.B. einen Zahnkranz oder irgendwelche Absätze oder Stege, die in radialer Richtung ineinander greifen. Der Verdrehanschlag kann dabei auch die Funktion einer Zentrierung übernehmen.

Bevorzugt ist die Montageeinrichtung sowohl für ein Sichern gegen Verdrehen, insbesondere mittels einer in axialer Richtung angeordneten Durchführung, als auch für ein Sichern des Verbindungsbauteils in axialer Richtung, insbesondere mittels mindestens einer in radialer Richtung angeordneten Durchführung, eingerichtet. Hierdurch kann das Nachjustieren oder Einstellen der Drehkupplung ohne weitere Hilfsmittel oder Abstützvorrichtungen auch problemlos dann erfolgen, wenn die Stativvorrichtung eine bedeutende Eigenmasse aufweist, z.B. wenn eine komplette Versorgungskonsole an der Stativvorrichtung befestigt ist.

Erfindungsgemäß weist die Justageeinrichtung eine Mehrzahl von Kupplungspunkten in Form von Löchern oder Durchführungen, jeweils zum Festlegen einer von einer Mehrzahl von Verdrehpositionen der Drehkupplung, auf, welche auf einem Teilkreis angeordnet sind, wobei die Justageeinrichtung eine ringförmige Geometrie aufweist oder als ringförmige Scheibe (Flachring) ausgebildet ist. Die Kupplungspunkte sind jeweils bevorzugt von einer Oberseite der Justageeinrichtung zugänglich. Der Teilkreis ist bevorzugt größer als ein Durchmesser der Kavität, was eine gute Zugänglichkeit von außen sicherstellen kann. Bevorzugt sind die Kupplungspunkte möglichst weit radial außen an der Justageeinrichtung angeordnet. Hierdurch kann das Einstellen auf einfache Weise erfolgen, selbst bei schwer zugänglicher Anordnung der Justageeinrichtung unter einer Raumdecke. Bevorzugt sind die Kupplungspunkte von einer Stirnseite der Justageeinrichtung zugänglich. Die Löcher oder Durchführungen sind bevorzugt in axialer Richtung ausgerichtet, insbesondere parallel zur Drehachse.

Die Justageeinrichtung weist bevorzugt einen Außendurchmesser auf, welcher größer gleich als die weiteren Komponenten der Montagevorrichtung ist. Hierdurch kann die Justageeinrichtung eine Schnittstelle an einer Umfangsfläche oder einem Außenrand bereitstellen, insbesondere Montageschlitze, an welcher eine Verkleidung oder Abdeckung der Montagevorrichtung montiert werden kann. Die Befestigung einer Verkleidung an der Justageeinrichtung hat den Vorteil, dass die Verkleidung auf einfache Weise entfernt werden kann und dass die Drehkupplung auf einfache Weise zugänglich ist.

Die Justageeinrichtung kann eine ringförmige Auflagefläche aufweisen, welche geometrisch korrespondierend zu einer ringförmigen Lagerfläche des Verbindungsbauteils ausgebildet ist. Hierdurch kann das Verbindungsbauteil auf exakte Weise mit der Justageeinrichtung gekuppelt werden. Die Auflagefläche kann dabei als Anschlag für einen korrespondierenden Absatz des Verbindungsbauteils dienen.

Gemäß einer Variante weist die Justageeinrichtung eine Durchführung mit einem Innendurchmesser kleiner dem Durchmesser der Kavität bzw. kleiner einem Innendurchmesser einer Innenmantelfläche der Montageeinrichtung auf. Hierdurch kann das Verbindungsbauteil an einem Absatz des Verbindungsbauteils an der Justageeinrichtung zur Anlage gebracht werden.

Gemäß einem Ausführungsbeispiel weist die Montageeinrichtung eine ebene untere Stirnfläche auf, an welcher die Justageeinrichtung in einer vordefinierbaren Axialposition zur Anlage bringbar ist. Hierdurch kann die Justageeinrichtung auf exakte Weise relativ zur Montageeinrichtung ausgerichtet werden, so dass das Verdrehsicherungselement in unterschiedlichen Positionen ohne Verkanten anordenbar ist, insbesondere auf manuelle Weise.

Gemäß einem Ausführungsbeispiel weist die Montageeinrichtung mindestens einen Befestigungsabschnitt mit einer Mehrzahl von Befestigungsmitteln, insbesondere Löchern oder Bohrungen auf, wobei die Befestigungsmittel unterschiedliche axiale Montagepositionen definieren. Hierdurch kann auf einfache Weise eine spezifische Höhenposition der Stativvorrichtung relativ zu einer Raumdecke bzw. einem Deckenflansch eingestellt werden.

Bevorzugt weist die Justageeinrichtung 15 bis 30, bevorzugt 20 bis 25 Löcher auf, so dass eine relative Drehposition der Spindel relativ zur Montageeinrichtung in vergleichsweise kleinen Winkelschritten eingestellt werden kann, z.B. in Schritten von 15°. Ein solches Einstellen der relativen Drehposition ist insbesondere in Hinblick auf die Drehbewegung begrenzende Anschläge oder Verdrehsicherungen der Stativvorrichtung vorteilhaft. Somit kann der Aktionsradius der Stativvorrichtung zum Positionieren der medizintechnischen Einrichtung auf flexible Weise eingestellt werden.

Gemäß einer Variante weist die Montageeinrichtung eine Mehrzahl von Gewindebohrungen zur Aufnahme von in Längsrichtung einbringbaren Befestigungselementen auf, wobei die Gewindebohrungen auf demselben Teilkreis angeordnet sind wie korrespondierende Kupplungspunkte/Löcher der Justageeinrichtung. Die Gewindebohrungen sind bevorzugt an einer unteren Stirnseite der Montageeinrichtung angeordnet und erstrecken sich zumindest annähernd in Längsrichtung. Dies ermöglicht eine einfache Montage und ein einfaches Nachjustieren. Bevorzugt sind drei bis fünf Gewindebohrungen vorgesehen, die im montierten Zustand der Montageinrichtung von einer Unterseite zugänglich sind, so dass Befestigungselemente in axialer Längsrichtung von unten eingesteckt und befestigt, insbesondere angeschraubt werden können. Ein Monteur kann dabei bei der Montage aller Befestigungselemente unter der Montageeinrichtung zumindest annähernd in derselben Position stehen bleiben. Eine Verschraubung in radialer Richtung ist nicht erforderlich. Dies ermöglicht auch eine Position des Monteurs weiter unten, und damit eine sicherere Montage mit weniger Risiken, z.B. weniger Risiko in Hinblick auf einen Sturz des Monteurs. Auch kann auf einfachere Weise eine Sichtprüfung der Befestigungselemente in Hinblick auf eine korrekte Position erfolgen. Ein möglicherweise in Montagerichtung, d.h. in Längsrichtung manuell aufzubringender Druck oder eine manuell aufzubringende Kraft kann leichter genau in der Längsrichtung aufgebracht werden.

Gemäß einem Ausführungsbeispiel ist der mindestens eine Befestigungsabschnitt an einer Außenmantelfläche durch einen in radialer Richtung hervorstehenden Steg gebildet, wobei die Montageeinrichtung bevorzugt mindestens drei Befestigungsabschnitte aufweist, welche sternförmig angeordnet sind, insbesondere in gleichem Abstand in Umfangsrichtung zueinander. Hierdurch kann eine Last (insbesondere Gewichtskraft oder ein Drehmoment) an einem weit außen liegenden Kraftangriffspunkt übertragen werden. Speziell bei mehreren symmetrisch um den Umfang der Montageeinrichtung verteilten Befestigungsabschnitten kann eine Kraft homogen verteilt an vorteilhaften Kraftangriffspunkten übertragen werden. Auch kann für den Fall, dass die Justageeinrichtung an der Montageeinrichtung befestigt werden soll, eine bestimmte Drehposition auf vergleichsweise genaue Weise oder in besonders kleinen Dreh-Schritten oder Dreh-Winkeln eingestellt werden, insbesondere unabhängig von einem Durchmesser der Spindel und weitgehend unabhängig von den zu übertragenden Lasten.

Bevorzugt sind die Befestigungsabschnitte umlaufend an der Außenmantelfläche angeordnet, insbesondere in demselben Abstand in Umfangsrichtung zueinander. Dies erleichtert ein Einstellen in Hinblick auf eine Vielzahl unterschiedlicher Drehwinkel-Positionen.

Erfindungsgemäß weist die Montageeinrichtung eine insbesondere rohrförmige/rohrartige Aufnahme für das Verdrehsicherungselement auf, wobei die Aufnahme bevorzugt an einer Außenseite, insbesondere Außenmantelfläche der Montageeinrichtung angeordnet ist oder die Außenmantelfläche zumindest abschnittsweise bildet. Hierdurch kann das Verdrehsicherungselement auf einfache Weise entfernt oder eingesetzt werden. Die Aufnahme weist bevorzugt eine Durchgangsbohrung auf, welche bis zu einer unteren Stirnseite der Montageeinrichtung führt. Eine derart ausgestaltete Aufnahme kann auch als Sicherungsrohr bezeichnet werden.

Bevorzugt ist die Aufnahme zur Anordnung eines in axialer Richtung ausgerichteten Verdrehsicherungselements ausgebildet, insbesondere einer Bolzenverbindung in axialer Richtung. Die Aufnahme umfasst dabei bevorzugt einen unteren (insbesondere rohrartigen) Aufnahmeabschnitt, welcher eine axiale Erstreckung kleiner einer Länge des Verdrehsicherungselements aufweist.

Gemäß einem Ausführungsbeispiel weist die Aufnahme einen Zugang auf, insbesondere einen Zugang von radial außen, wobei der Zugang bevorzugt in Form einer Ausfräsung ausgebildet ist. Hierdurch wird eine manuelle Justage erleichtert. Bevorzugt weist der Zugang in axialer Richtung eine Erstreckung auf, welche größer ist als eine Länge des Verdrehsicherungselements und größer als eine axiale Erstreckung eines unteren Abschnitts der Aufnahme.

Der Zugang weist bevorzugt eine oberseitige Zugangsfläche auf, an welcher das Verdrehsicherungselement zur Anlage gebracht werden kann, insbesondere in einer vordefinierten Axialposition. An der oberseitigen Zugangsfläche kann z.B. ein Bolzen mit einem Rand/Kopf/Absatz gelagert werden, so dass die Drehkupplung auf einfache Weise manuell eingestellt werden kann. Der Bolzen kann in die Aufnahme gesteckt werden und allein durch Gravitationskräfte darin gesichert werden. Hierdurch muss ein Monteur nur eine Steckbewegung ausführen, welche auch in besonders kurzer Zeit ausgeführt werden kann. Eine Drehbewegung oder ein Verschrauben ist nicht erforderlich, was insbesondere an schlecht zugänglichen Positionen direkt unterhalb einer Raumdecke vorteilhaft ist. Das Verdrehsicherungselement kann manuell auf einfache Weise demontiert und wieder montiert werden, insbesondere werkzeuglos. Dies liefert gerade an schwer zugänglichen Aufhängungspunkten der Stativvorrichtung Vorteile, nicht zuletzt weil ein Monteur beide Hände frei hat. Gemäß einer Variante kann das Verdrehsicherungselement nicht nur durch dessen Schwerkraft/Gravitationskraft gesichert werden, sondern alternativ oder zusätzlich auch durch einen Federstecker.

Gemäß einem Ausführungsbeispiel weist die Montagevorrichtung eine Axialsicherung auf, mittels welcher das Verbindungsbauteil in einer vordefinierten Axialposition an der Montageeinrichtung lagerbar ist, insbesondere relativ verdrehbar zur Montageeinrichtung. Die Axialsicherung kann die Montage oder auch das Nachjustieren erleichtern. Das Stativ kann mittels der Axialsicherung gesichert werden, insbesondere während der Justage der Drehposition, oder während dem Befestigen von einzelnen Befestigungselementen. Die Montagevorrichtung kann auch ein Risiko eines Verkantens innerhalb der Montageeinrichtung vermindern. Die Montagevorrichtung kann dabei mit nur drei Hauptkomponenten eine auf einfache Weise einstellbare Drehkupplung bereitstellen. Die Kupplung besteht dabei aus drei Hauptkomponenten, nämlich der Montageeinrichtung, der Justageeinrichtung bzw. dem Flachring sowie der Axialsicherung. Dabei kann die Axialsicherung dauerhaft montiert sein bzw. bleiben.

Bevorzugt bildet die Axialsicherung in Verbindung mit der Kavität ein axialfestes Drehlager für das Verbindungsbauteil, also ein Lager, bei welchem eine Verdrehung in einer vordefinierten Axialposition möglich ist. Das axialfeste Drehlager liefert dabei einen Bewegungsfreiheitsgrad um die Drehachse und verhindert eine Bewegung entlang der Drehachse.

Gemäß einer Variante ist die Axialsicherung eingerichtet, dauerhaft montiert zu sein und die axiale Sicherung des Verbindungsbauteils in unterschiedlichen bzw. allen Montagesituationen sicherzustellen.

Gemäß einem Ausführungsbeispiel ist die Montageeinrichtung zum Halten und verdrehbaren Lagern des Verbindungsbauteils in einer vorbestimmten axialen Relativposition ausgebildet, wobei an einer die Kavität begrenzenden Innenmantelfläche der Montageeinrichtung eine Fase, Kante oder Einfräsung vorgesehen ist. Hierdurch kann durch einfach aufgebaute konstruktive Komponenten ein axialfestes Drehlager gebildet werden, welches bei einer Montage der Stativvorrichtung auf einfache Weise montierbar ist. Die Innenmantelfläche der Montageeinrichtung kann dabei zumindest abschnittsweise zylindrisch sein. Bevorzugt weist die Innenmantelfläche eine oder mehrere Fasen oder Abstufungen auf, welche eingerichtet sind, eine Spindel innerhalb der Montageeinrichtung zu zentrieren und/ in einer vordefinierten Axialposition zu positionieren. Bei einer vordefinierten Axialposition kann die Montage mehr oder weniger "blind" erfolgen, was insbesondere bei schlecht zugänglichen Deckenstativen von Vorteil ist. Derartige Fasen oder Abstufungen können auch die Montage erleichtern, insbesondere bei einer vergleichsweise weichen Montageeinrichtung (z.B. aus Aluminium-Material), in welcher eine vergleichsweise harte Spindel (z.B. aus einem StahlMaterial) gelagert ist. Derartige Fasen oder Abstufungen können z.B. ein Verkanten oder das Ausbilden von Graten verhindern. Bevorzugt sind die Fasen oder Abstufungen in Umfangsrichtung angeordnet, insbesondere umlaufend.

Die Fasen oder Abstufungen sind in axialer Richtung bevorzugt in einem vergleichsweise großen Abstand voneinander vorgesehen, insbesondere in Abhängigkeit von einer bestimmten Anzahl, und insbesondere gleichmäßig verteilt entlang der Montageeinrichtung. Bei einer derartigen Ausgestaltung kann die Innenmantelfläche auch als eine Mehrzahl von Zentrierflächenabschnitten ausgebildet sein. An der Spindel sind bevorzugt geometrisch korrespondierende Fasen oder Abstufungen ausgebildet. Einer oder mehrere Zentrierflächenabschnitte ermöglichen nicht zuletzt eine freiere Auswahl eines geeigneten Materials für die Spindel und/oder für die Montageeinrichtung.

Gemäß einer Variante kann die Montage auch durch unterschiedlich große Passungsdurchmesser vereinfacht werden.

Gemäß einem Ausführungsbeispiel umfasst die Axialsicherung eine tangential an der Montageeinrichtung angeordnete Durchführung, welche eine Außenmantelfläche der Montageeinrichtung durchstößt, bevorzugt an zwei Punkten, und welche eine/die Innenmantelfläche der Montageeinrichtung schneidet. Die Durchführung (insbesondere Bohrung oder Ausfräsung) kann wie eine Sekante relativ zur Außenmantelfläche der Montageeinrichtung angeordnet sein. In die Durchführung kann ein tangential zum Verbindungsbauteil angeordnetes Sicherungselement (insbesondere Bolzen oder Riegel) eingebracht werden, welches in eine Nut des Verbindungsbauteils eingreifen kann und das Verbindungsbauteil axial in der Montageeinrichtung sichern kann. Bevorzugt ist das Sicherungselement tangential zur Montageeinrichtung anordenbar und geometrisch korrespondierend zur Durchführung ausgebildet, insbesondere an einer unterseitigen Kontaktfläche, was eine robuste Abstützung sicherstellen kann. Bei einem tangentialen Riegel kann eine spezifische Geometrie mit einem oder mehreren Absätzen realisiert werden, und bei einem tangentialen Bolzen kann die Konstruktion auf einfache Weise, insbesondere auch in Hinblick auf geringe Kosten, ausgelegt werden.

Die tangential ausgerichtete Durchführung kann eine Innenmantelfläche der Kavität derart schneiden, dass der Riegel weiter innen angeordnet ist als die Innenmantelfläche. Beispielsweise ragt der Riegel um die Hälfte seines Durchmessers weiter nach innen als die Innenmantelfläche. Eine als Sekante angeordnete Durchführung liefert den Vorteil eines z.B. im Vergleich zu einem radial ausgerichteten Sicherungsbolzens vergleichsweise langen Eingriffsabschnitt des Riegels mit dem Verbindungsbauteil. Der Riegel ist dabei tangential zum Verbindungsbauteil angeordnet und greift an einer Umfangsseite des Verbindungsbauteils abschnittsweise tangential in das Verbindungsbauteil ein. Diese Art einer Axialsicherung hat auch den Vorteil, dass die Axialsicherung nicht entfernt werden muss, insbesondere beim Verdrehen des Verbindungsbauteils während der Montage. Eine Reibung bei relativer Drehung des Verbindungsbauteils innerhalb der Kavität kann dabei vergleichsweise klein gehalten werden. Ein Verdrehen kann selbst bei langen Tragarmen bzw. hohen Gewichten oder Momenten auf einfache Weise erfolgen, insbesondere durch Eingriff in eine Aussparung an der Unterseite der Justageeinrichtung. Die Axialsicherung bzw. der Tangential-Riegel kann in der tangentialen Position positioniert bleiben.

Gemäß einer Variante können zwischen der Justageeinrichtung und der unteren Stirnseite der Montageeinrichtung Distanzscheiben vorgesehen sein, um eine Höhenposition des Verbindungsbauteils relativ zur Montageeinrichtung einstellen zu können. Auch können mehrere tangentiale Durchführungen jeweils zur Aufnahme eines Sicherungselements zur Axialsicherung übereinander in der Montageeinrichtung vorgesehen sein. Hierdurch kann auf einfache Weise eine Höhenverstellung erfolgen, wobei Distanzscheiben nicht notwendigerweise erforderlich sind.

Bevorzugt ist an der Durchführung eine Auflagefläche ausgebildet, welche eingerichtet ist, eine vom Verbindungsbauteil ausgeübte (Gewichts-)Kraft auf die Montageeinrichtung zu übertragen. Gemäß einer Variante ist die Auflagefläche U-förmig. Hierdurch lassen sich die Durchführungen auf wirtschaftliche Weise herstellen, insbesondere durch Fräsen. Andererseits können sowohl in der Montageeinrichtung als auch im Verbindungsbauteil Kerbspannungen minimal gehalten werden. Bevorzugt weist die Durchführung auf der oberen Seite kleinere Radien auf als auf der Unterseite. Dadurch kann vorgegeben werden, in welcher Anordnung das jeweilige Sicherungselement in der Durchführung angeordnet werden soll. Ein Risiko einer fehlerhaften Montage kann dadurch verringert werden.

Gemäß einem Ausführungsbeispiel umfasst die Axialsicherung mindestens ein Sicherungselement, insbesondere einen Riegel oder Bolzen, welches ausgelegt und eingerichtet ist, die Gewichtskraft der Stativvorrichtung vom Verbindungsbauteil auf die Montageeinrichtung zu übertragen. Hierdurch kann ein Nachjustieren der Drehposition erfolgen, ohne irgendwelche Komponenten der Stativvorrichtung, insbesondere irgendwelche Komponenten einer Zentralachse, demontieren zu müssen. Die gesamte Stativvorrichtung kann während des Einstellens der Drehposition an der Axialsicherung gelagert werden. Mit anderen Worten ist die Axialsicherung eingerichtet, ein Drehlager für die Stativvorrichtung zu bilden. Dies erleichtert das Nachjustieren erheblich, denn lediglich die Justageeinrichtung braucht gelöst und wieder in einer nachjustierten Drehposition befestigt zu werden.

Gemäß einem Ausführungsbeispiel sind die Durchführung und die Aufnahme für das Verdrehsicherungselement an demselben Umfangsflächenabschnitt der Montageeinrichtung angeordnet, insbesondere von derselben Seite manuell zugänglich. Dies erleichtert das Einstellen und Montieren der Vorrichtung. Dabei kann eine Sicherung des Verdrehsicherungselements und der Sicherungselemente zur Axialsicherung mittels Federsteckern erfolgen, und zwar von derselben Seite bzw. am selben Umfangsflächenabschnitt. Dies liefert nicht zuletzt Vorteile bei der Montage.

Gemäß einem Ausführungsbeispiel ist die Montageeinrichtung ein rohrartiges Stranggussteil, insbesondere aus Aluminium, wobei die Montageeinrichtung bevorzugt einstückig ist. Diese Ausgestaltung ermöglicht zum einen eine hohe Stabilität, zum anderen auch eine kostengünstige Herstellungsweise. Dabei kann die axiale Erstreckung um die Drehachse weitgehend frei gewählt werden, je nach Einsatzbedingungen und Montageposition. Bevorzugt ist auch ein Halter zum Verbinden der Montageeinrichtung mit einem Deckenflansch als Stranggussteil ausgebildet.

Gemäß einer Variante sind keine Halter vorgesehen. Die Montageeinrichtung weist dabei bevorzugt Schraubkanäle auf, insbesondere am Rand, mittels welchen die Montageeinrichtung direkt an der/einer Flanschplatte montierbar ist. Hierdurch kann nicht zuletzt eine besonders kostengünstige Variante bereitgestellt werden. Die Anzahl von Komponenten oder Teilen ist reduziert. Die Montage kann weiter vereinfacht werden, insbesondere bei solchen Anwendungen, welche keine besonders große Flexibilität in Hinblick auf Höhenverstellung erfordern.

Gemäß einem Ausführungsbeispiel weist die Montagevorrichtung zwei unterschiedlich große Passflächen auf, welche korrespondierend zu Passflächen des Verbindungsbauteils ausgebildet sind und eingerichtet ist zur Montagevereinfachung und Zweipunktlagerung des Verbindungsbauteils. Dies hat Vorteile in Hinblick auf eine sichere Montage und eine belastbare Lagerung.

Gemäß einem Ausführungsbeispiel weist die Justageeinrichtung eine Mehrzahl von Löchern jeweils zum Festlegen einer Verdrehposition auf, welche auf einem Teilkreis angeordnet sind, wobei die Justageeinrichtung ringförmig ist, wobei die Montageeinrichtung eine rohrartige Aufnahme für ein Verdrehsicherungselement aufweist, wobei die Aufnahme an einer Außenmantelfläche der Montageeinrichtung angeordnet ist, wobei die Aufnahme einen Zugang von radial außen aufweist, wobei der Zugang eine oberseitige Zugangsfläche aufweist, an welcher ein Verdrehsicherungselement zur Anlage gebracht werden kann, wobei die Justageeinrichtung eine Nut oder Feder aufweist, welche geometrisch korrespondierend zu einem am Verbindungsbauteil angeordneten Verdrehanschlag ausgebildet ist, wobei die Montagevorrichtung eine Axialsicherung aufweist, welche eine an einer die Kavität begrenzenden Innenmantelfläche der Montageeinrichtung vorgesehene Fase, Kante oder Einfräsung umfasst, wobei die Axialsicherung eine tangential an der Montageeinrichtung angeordnete Durchführung umfasst, welche eine Außenmantelfläche der Montageeinrichtung durchstößt und eine Innenmantelfläche der Montageeinrichtung schneidet. Durch diese Ausgestaltung lassen sich eine Vielzahl der mit der vorliegenden Erfindung erzielbaren Vorteile verwirklichen.

Vorteilhaft ist die Justageeinrichtung flach-ringförmig ausgebildet und weist in Bezug auf die Anordnung der Kupplungspunkte bzw. Löcher eine wenigstens 12-zählige Rotationssymmetrie, insbesondere eine wenigstens 24-zählige Rotationssymmetrie auf. D.h., dass benachbarte Kupplungspunkte bzw. Löcher bezogen auf ihr Zentrum bei der wenigstens 12-zähligen Rotationssymmetrie in einem Winkelabstand von höchstens 30° zueinander liegen, und dass benachbarte Kupplungspunkte bzw. Löcher bezogen auf ihr Zentrum bei der wenigstens 24-zähligen Rotationssymmetrie in einem Winkelabstand von höchstens 15° zueinander liegen.

Die zuvor beschriebene Aufgabe wird auch gelöst durch ein Montagesystem mit den Merkmalen des Anspruchs 13. Entsprechend wird ein Montagesystem mit einer erfindungsgemäßen Montagevorrichtung vorgeschlagen, wobei das Montagesystem das Verbindungsbauteil in Form einer Spindel sowie ein Verdrehsicherungselement und mindestens ein Sicherungselement zur Axialsicherung aufweist, wobei an der Spindel eine umlaufende Nut oder ein umlaufender Absatz vorgesehen ist, welche/r geometrisch korrespondierend zum Sicherungselement ausgebildet ist. Mit anderen Worten umfasst das Montagesystem unterschiedliche Sicherungselemente jeweils zur Axialsicherung und zur Verdrehsicherung. Bevorzugt sind beide Typen der Sicherungselemente an derselben Umfangsposition der Montageeinrichtung montierbar. Dabei kann ein einziges Sicherungselement zur Axialsicherung vorgesehen sein. Bevorzugt sind zwei Sicherungselemente zur Axialsicherung vorgesehen. Wahlweise können auch drei Sicherungselemente zur Axialsicherung vorgesehen sein.

Bevorzugt ist die Nut umlaufend vorgesehen und erstreckt sich orthogonal zur Längsrichtung. Eine umlaufende Nut liefert den Vorteil, dass der Bolzen der Axialsicherung unabhängig von einer spezifischen Drehposition in Eingriff mit der Spindel gebracht werden kann. Auch kann die Spindel auf einfache Weise relativ zur Montageeinrichtung gedreht werden, selbst bei einer auf die Axialsicherung in axialer Richtung einwirkenden Last. Dies erleichtert das Nachjustieren oder Umstellen der Drehposition.

Bevorzugt ist die Nut in einem Abstand zu einer Stirnseite oder einem stirnseitigen Anschlag der Spindel angeordnet, welcher in Längsrichtung gesehen einem Abstand der Bohrung von einem Gegenanschlag in der Kavität entspricht. Dies erleichtert die Montage, insbesondere da die Spindel mit der Stirnseite auf einfache Weise an einem/dem Gegenanschlag der Montageeinrichtung derart zur Anlage gebracht werden kann, dass die Spindel in der richtigen Axialposition angeordnet ist, um die Axialsicherung vorzusehen. In dieser Axialposition kann der Bolzen tangential an einer Außenmantelfläche der Spindel eingreifen. Ein Nachjustieren der Axialposition ist nicht erforderlich. Der Gegenanschlag der Montageeinrichtung kann dabei auch durch einen Boden der Kavität oder einen umlaufenden ringförmigen oder scheibenförmigen Gegenanschlag am Boden der Kavität bereitgestellt werden.

Gemäß einer Variante ist an der Spindel ein Absatz ausgebildet, welchen die Justageeinrichtung (in montiertem Zustand) in radialer Richtung überlappt. Dies ermöglicht eine Lagerung der Spindel mittels der Justageeinrichtung. Dieser Absatz kann ermöglichen, die Justageeinrichtung zusammen einem oder mehreren Tragarmen über eine die Tragarme sichernde untere Wellenmutter zu fixieren. Die Montage kann insbesondere dadurch erleichtert werden, dass die Justageeinrichtung vor dem Einschieben der Spindel in die Montageeinrichtung gesichert wird, speziell in Hinblick auf ein Verrutschen nach unten.

Zur Montage kann zunächst die Montageeinrichtung an der Raumdecke montiert werden. Dann kann die Spindel von unten in die Kavität der Montageeinrichtung gesteckt werden. Die Justageeinrichtung ist dabei bevorzugt bereits auf der Spindel angeordnet und kann an der Montageeinrichtung befestigt werden. Hierdurch kann auch die Spindel in axialer Längsrichtung an der Montageeinrichtung positioniert werden. Wahlweise kann die Spindel vor dem Befestigen der Justageeinrichtung auch mittels einer Axialsicherung an der Montageeinrichtung gesichert werden, so dass die Justageeinrichtung in einer bestimmten Drehposition positioniert werden kann, ohne dass gleichzeitig eine Gewichtskraft der Spindel mittels der Justageeinrichtung aufgenommen werden muss. Dies erleichtert die Montage oder auch ein nachträgliches Einstellen einer bestimmten Drehposition.

Gemäß einer Variante hat der Absatz der Spindel einen Außendurchmesser, der größer ist als der Innendurchmesser einer Durchführung der Justageeinrichtung. Bei dieser Ausgestaltung ist die Spindel wahlweise auch mittels der Justageeinrichtung in axialer Richtung lagerbar. Die Überlappung in radialer Richtung beträgt bevorzugt mindestens 1mm.

Gemäß einer Variante ist unterhalb des Absatzes ein Verdrehanschlag, insbesondere eine (Paß-)Feder, angeordnet, der/die bevorzugt in Längsrichtung ausgerichtet ist. Der Verdrehanschlag ist geometrisch korrespondierend zu einem Verdrehanschlag der Justageeinrichtung ausgebildet. Der Verdrehanschlag kann z.B. eine in einer entsprechenden Nut auf der Spindel montierte Paßfeder sein, oder eine angegossene Feder.

Gemäß einem Ausführungsbeispiel umfasst das Montagesystem ein Verdrehsicherungselement in Form eines in axialer Richtung eingreifenden Bolzens sowie ein oder zwei Sicherungselemente in Form von in tangentialer Richtung eingreifenden Riegeln, wobei der Bolzen und die Riegel angrenzend an einen in einer Aufnahme für das Verdrehsicherungselement vorgesehenen Zugang angeordnet sind. Diese Anordnung erleichtert das Einstellen und die Montage. Der tangentiale Riegel kann wahlweise auch als Bolzen ausgeführt sein.

Die zuvor beschriebene Aufgabe wird auch gelöst durch eine Justageeinrichtung für eine im Operationssaal anordenbare Stativvorrichtung zum örtlichen Positionieren oder Verlagern einer medizintechnischen Einrichtung, insbesondere durch eine Drehbewegung, wobei die Justageeinrichtung in einer vordefinierten Drehwinkelposition an einem Verbindungsbauteil der Stativvorrichtung anordenbar ist, wobei die Justageeinrichtung eingerichtet ist, eine Mehrzahl von Kupplungspunkten, insbesondere Löcher oder Durchführungen, jeweils zum Festlegen einer Verdrehposition aufweist, wobei die Justageeinrichtung als ringförmige Scheibe ausgebildet ist. Damit ergeben sich bereits zuvor beschriebene Vorteile.

Die zuvor beschriebene Aufgabe wird auch gelöst durch eine Montageeinrichtung für eine im Operationssaal anordenbare Stativvorrichtung zum örtlichen Positionieren oder Verlagern einer medizintechnischen Einrichtung, insbesondere durch eine Drehbewegung, wobei sich die Montageeinrichtung in einer Längsrichtung entlang einer Drehachse erstreckt und eine in Längsrichtung ausgerichtete, insbesondere zylindrische Kavität zur Aufnahme eines drehbar lagerbaren Verbindungsbauteils der Stativvorrichtung aufweist, wobei die Montageeinrichtung eine von außen zugängliche Aufnahme für ein Verdrehsicherungselement zum Festlegen einer Verdrehposition aufweist, wobei die Montageeinrichtung eingerichtet ist, mittels des Verdrehsicherungselements ein Drehmoment zwischen dem Verbindungsbauteil und einer Flanschplatte zu übertragen. Damit ergeben sich bereits zuvor beschriebene Vorteile.

Die zuvor beschriebene Aufgabe wird auch gelöst durch die Verwendung gemäß Anspruch 14 der erfindungsgemäßen Montagevorrichtung zum örtlichen Positionieren oder Verlagern einer medizintechnischen Einrichtung, insbesondere durch eine Drehbewegung, wobei die Montagevorrichtung eine einstellbare Verdrehposition eines Verbindungsbauteils der Stativvorrichtung relativ zu einer Flanschplatte festlegt, mittels welcher die Stativvorrichtung im Operationssaal befestigt ist. Hierdurch ergeben sich bereits zuvor beschriebene Vorteile.

In den nachfolgenden Zeichnungsfiguren wird die Erfindung anhand von Ausführungsbeispielen näher erläutert. Es zeigen:
- Figur 1: in perspektivischer Seitenansicht eine Montagevorrichtung gemäß einem Ausführungsbeispiel der Erfindung in einer montierten Anordnung an einer Stativvorrichtung;
- Figur 2: in perspektivischer geschnittener Seitenansicht die in Fig. 1 gezeigte Montagevorrichtung;
- Figur 3: in perspektivischer Seitenansicht eine Justageeinrichtung der in der Fig. 1 gezeigten Montagevorrichtung in Draufsicht, wobei die Justageeinrichtung um eine Spindel der Stativvorrichtung angeordnet ist;
- Figur 4: in Draufsicht von einer Unterseite aus betrachtet einzelne Komponenten der in Fig. 1 gezeigten Montagevorrichtung;
- Figur 5: in perspektivischer Seitenansicht einzelne Komponenten der in Fig. 1 gezeigten Montagevorrichtung;
- Figur 6: in perspektivischer Seitenansicht eine Montageeinrichtung der in Fig. 1 gezeigten Montagevorrichtung;
- Figur 7: in perspektivischer Ansicht von einer Unterseite die in der Fig. 1 gezeigte Justageeinrichtung;
- Figur 8: in perspektivischer Ansicht von oben die in der Fig. 7 gezeigte Justageeinrichtung;
- Figur 9: in perspektivischer Ansicht die in der Fig. 3 gezeigte Spindel;
- Figur 10: in perspektivischer Ansicht ein Sicherungselement der in Fig. 1 gezeigten Montagevorrichtung;
- Figur 11: in perspektivischer Ansicht eine Montagevorrichtung gemäß einem weiteren Ausführungsbeispiel der Erfindung in einer montierten Anordnung an einer Stativvorrichtung mit montierten Sicherungselementen;
- Figur 12: in perspektivischer Ansicht ein Verdrehsicherungselement, welches zur Sicherung einer Drehposition der Montagevorrichtung eingerichtet ist;
- Figur 13: in geschnittener Seitenansicht ein weiteres Ausführungsbeispiel einer Montagevorrichtung; und
- Figur 14: eine Spindel speziell für die in Figur 13 gezeigte Montagevorrichtung.

Im Zusammenhang mit der Beschreibung der folgenden Figuren wird bei Bezugszeichen, falls sie bei einzelnen Figuren nicht explizit erläutert werden, auf die weiteren Figuren verwiesen.

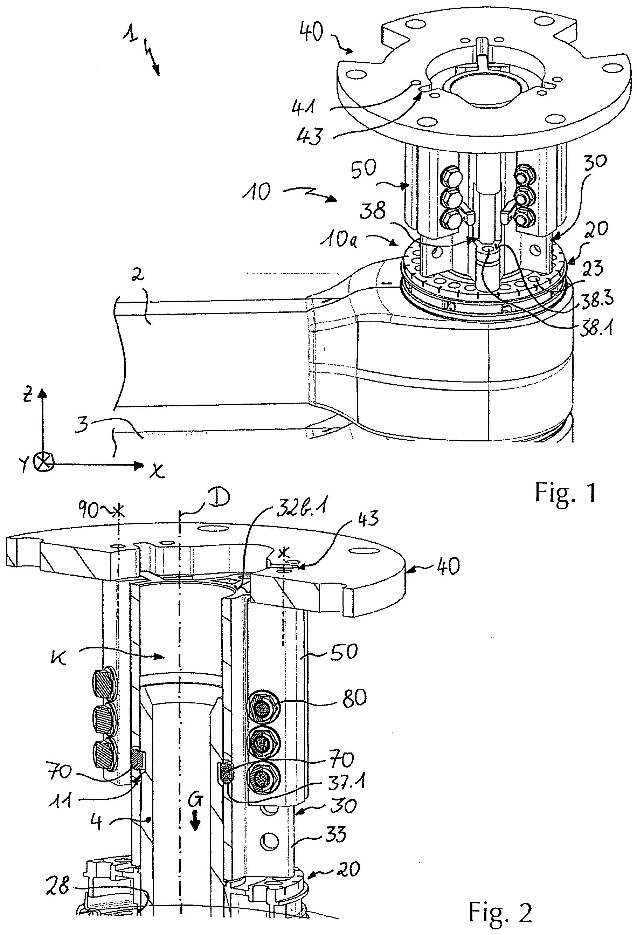

In der Figur 1 ist eine Stativvorrichtung für eine Deckenmontage gezeigt. Die Stativvorrichtung 1 umfasst einen ersten Träger 2 und einen zweiten Träger 3, welche drehbar um eine Drehachse übereinander in einem Drehgelenk gelagert sind. Für die Montage der Stativvorrichtung 1 an einer (Zwischen-)Decke sind die beiden Träger 2, 3 mit einer Montagevorrichtung 10 verbunden bzw. indirekt verkuppelt, welche einen anpassbaren Mechanismus 10a in Form einer einstellbaren Drehkupplung aufweist. Die Stativvorrichtung 1 kann mittels einer Flanschplatte 40 an einer Decke oder Zwischendecke montiert werden. Die Flanschplatte 40 weist Öffnungen 41, insbesondere Bohrungen auf, durch welche hindurch Befestigungsmittel, wie z.B. Schrauben montierbar sind. Die Montage erfolgt mittels Haltern 50, welche eine Verbindung der Flanschplatte 40 zu einer Montageeinrichtung 30 sicherstellen. Die Montageeinrichtung 30 wirkt mit einer Justageeinrichtung 20 zusammen. Die Justageeinrichtung 20 kann als Flachring beschrieben werden. Hierzu weist die Montageeinrichtung 30 eine Verdrehsicherung auf, insbesondere eine Aufnahme 38 in Form eines rohrartigen Abschnitts zur Aufnahme eines Sicherungselements. An einer oberseitigen Zugangsfläche 38.3 kann durch eine Durchgangsbohrung 38.1 ein Bolzen oder Stift gesteckt werden, welcher mit einem spezifischen Kupplungspunkt 23 der Justageeinrichtung zusammenwirkt. Mehrere Kupplungspunkte 23 definieren dabei jeweils eine spezifische Verdrehposition. Die Kupplungspunkte 23 können z.B. als Löchern oder Bohrungen oder seitlich offene Schlitze oder Aussparungen ausgebildet sein.

Dabei deutet ein Koordinatensystem die horizontale und bei einer Deckenmontage gleichzeitig auch radiale x-Richtung sowie die vertikale z-Richtung an.

Die Justiereinrichtung 20 kann dabei z.B. auf einem Innenring eines im Träger 2 angeordneten Kugellagers auf dem Träger 2 gelagert sein.

In der Figur 2 ist gezeigt, auf welche Weise die Flanschplatte 40 mit der Montageeinrichtung 30 verbunden werden kann. Die Flanschplatte 40 weist mehrere Radialnuten 43 auf, welche geometrisch korrespondierend zu Befestigungsabschnitten 33 der Montageeinrichtung 30 ausgebildet sind. Dabei kann die Flanschplatte 40 mittels Befestigungselementen 90, insbesondere Schrauben, so an den Haltern 50 befestigt werden, dass eine Gewichtskraft G von einem in der Montageeinrichtung 30 gelagerten Verbindungsbauteil 4, insbesondere einer Spindel, über die Montageeinrichtung 30, die Halter 50 und die Befestigungselemente 90 auf die Flanschplatte 40 und damit auf eine Decke oder Zwischendecke übertragen werden kann. Die Spindel 4 ist in der durch die Montageeinrichtung 30 gebildeten Kavität K gelagert. Dabei ist eine relative Verdrehung der Spindel 4 relativ zur Montageeinrichtung 30 möglich. Die gezeigte Anordnung ist um eine sich in z-Richtung erstreckende Drehachse D drehbar gelagert. Die relative Verdrehbarkeit kann mittels der Justageeinrichtung 20 verhindert werden.

Eine axiale Relativbewegung bzw. Verlagerung nach unten wird dadurch verhindert, dass eine Axialsicherung 11 vorgesehen ist, an welcher die Spindel 4 zur Anlage kommt. Im gezeigten Beispiel ist die Axialsicherung 11 durch mehrere Sicherungselemente 70, insbesondere Riegel oder Bolzen, gebildet, welche in tangentialer Richtung in Bezug auf eine Drehachse D angeordnet sind und sowohl in die Spindel 4 als auch in die Montageeinrichtung 30 eingreifen.

Die Montageeinrichtung 30 kann in unterschiedlichen axialen Relativpositionen (Höhenpositionen) relativ zu den Haltern 50 bzw. zur Flanschplatte 40 montiert werden, nämlich mittels Befestigungselementen 80, insbesondere Schrauben, welche in entsprechende Löcher oder Durchgangsbohrungen am jeweiligen Befestigungsabschnitt 33 bzw. am jeweiligen Halter 50 eingreifen.

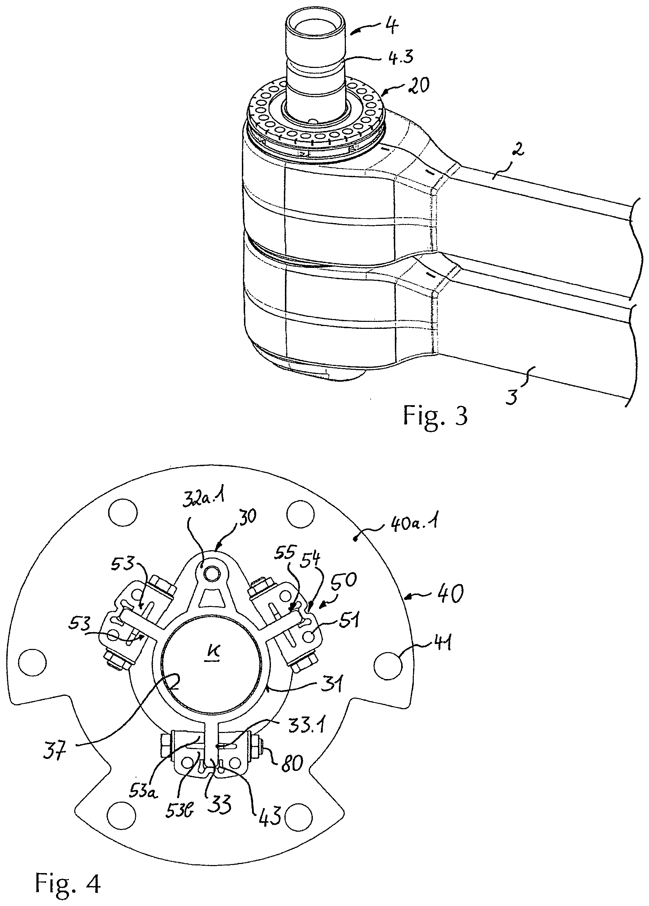

In der Figur 3 ist die Spindel 4 in einer Form gezeigt, in welcher sie von unten in die Kavität K der Montageeinrichtung 30 eingesetzt werden kann. Dabei können die zuvor beschriebenen Sicherungselemente 70 in der Endposition mit einer umlaufenden Nut 4.3 zusammenwirken. Die Spindel 4 ist dann an der Nut 4.3 verdrehbar und axialfest in der Kavität K gelagert.

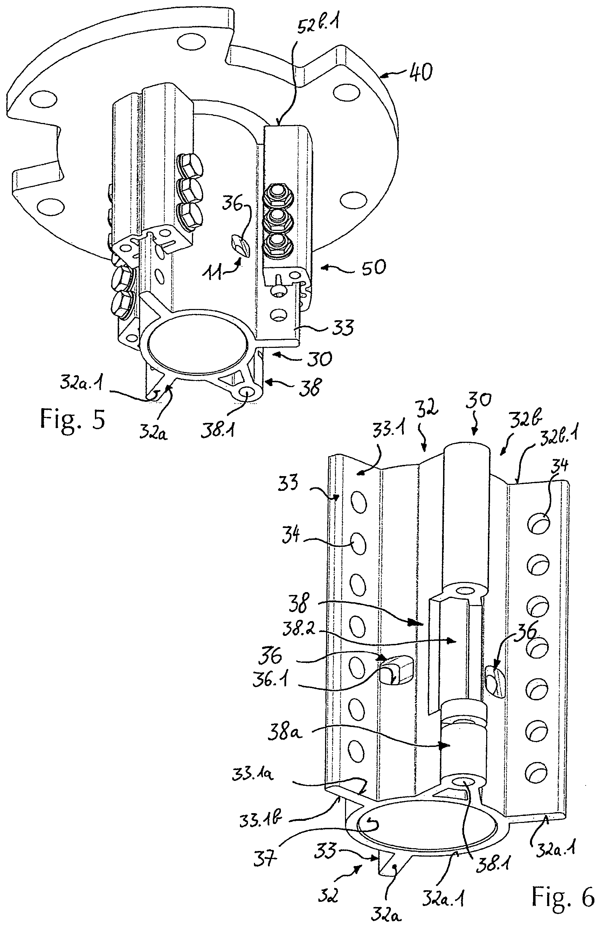

In der Figur 4 ist eine ebene unterseitige Stirnfläche 42a.1 der Flanschplatte 40 gezeigt, an welcher der jeweilige Halter 50 mit einer ebenen oberseitigen Stirnfläche 52.b.1 (angedeutet in Figur 5) zur Anlage kommen kann. Der jeweilige Halter 50 weist durchgehende Befestigungsmittel 51 auf, insbesondere Innengewindebohrungen, in welche z.B. Schrauben eingreifen können. Der jeweilige Befestigungsabschnitt 33 kommt an gegenüberliegenden Backen 53 zur Anlage. Die Backen können mittels der Schrauben 80 an eine entsprechende Radialflanke 33.1 gepresst werden. Jede Backe 53 weist einzelne Backenabschnitte 53a, 53b auf, welche eine sichere oder belastbare Verbindung des jeweiligen Halters 50 mit der Montageeinrichtung 30 sicherstellen können. Ferner weist der jeweilige Halter 50 eine Wellung 54 auf, welche eine nach außen weisenden Fläche zumindest abschnittsweise bildet, und dank welcher der Halter 50 auf vergleichsweise einfache bzw. flexible Weise aufgebogen und problemlos am jeweiligen Befestigungsabschnitt 33 montiert werden kann. Die gegenüberliegenden Backen 53 definieren eine Radialkavität 55, insbesondere in Schlitzform, welche geometrisch korrespondierend zum jeweiligen Befestigungsabschnitt 33 ausgebildet ist. Dabei erstrecken sich die Befestigungsabschnitte 33 in radialer Richtung nach außen von einer zylindrischen Außenmantelfläche 31 und bilden diese Außenmantelfläche weiter fort. Die Montageeinrichtung 30 weist eine zumindest abschnittsweise zylindrische Innenmantelfläche 37 auf, durch welche die Kavität K definiert bzw. in radialer Richtung begrenzt ist.

In der Figur 5 ist die Montageeinrichtung 30 in einem an der Flanschplatte 40 montierten Zustand von einer Unterseite gezeigt. Sowohl die Aufnahme 38 für eine Verdrehsicherung als auch die einzelnen Befestigungsabschnitte 33 definieren eine untere Stirnseite 32a, welche durch eine ebene untere Stirnfläche 32a.1 gebildet ist. An dieser Stirnfläche 32a.1 kann die Justageeinrichtung zur Anlage kommen. Die Montageeinrichtung 30 kann dabei z.B. als Stranggussprofil ausgebildet sein. An der Mantelfläche der Montageeinrichtung 30 sind eine oder mehrere Durchführungen 36, insbesondere in tangentialer Richtung, eingebracht, welche einen Teil der Axialsicherung 11 bilden.

In der Figur 6 ist die Montageeinrichtung 30 isoliert dargestellt. Die Montageeinrichtung 30 ist als Stranggussprofil ausgebildet und weist gegenüberliegende Stirnseiten 32 auf. Ebenso wie die untere Stirnseite 32a ist eine obere Stirnseite 32b durch eine ebene obere Stirnfläche 32b.1 gebildet. Entlang eines jeden Befestigungsabschnitts 33 sind eine Vielzahl von Löchern oder Befestigungsmitteln 34 vorgesehen, z.B. Gewindebohrungen oder Bohrungen. Die Durchführungen 36 definieren jeweils eine Auflagefläche 36.1, auf welcher das in Fig. 2 gezeigte Sicherungselement 70 zur Anlage kommen kann. Die Auflagefläche 36.1 kann dabei zumindest teilweise auch durch eine an der Innenmantelfläche 37 gebildete Fase, Kante oder Einfräsung 37.1 gebildet sein (vgl. Fig. 2). Ein Kraftflusspfad einer zu übertragenden Gewichtskraft verläuft durch diese Auflageflächen 36.1. Die Durchführungen 36 grenzen radial innen an mindestens zwei der Befestigungsabschnitte 33 an. Jeder Befestigungsabschnitt 33 weist gegenüberliegende, insbesondere parallel zueinander ausgerichtete Radialflanken 33.1a, 33.1b auf. Die Verdrehsicherung 38 bzw. die Aufnahme ist in Form eines Sicherungsrohrabschnitts ausgebildet. Die Verdrehsicherung 38 weist einen unteren rohrartigen Aufnahmeabschnitt 38a auf. Der untere Aufnahmeabschnitt 38a kann unterteilt sein, insbesondere durch einen Schlitz, wodurch ein zusätzliches Sichern eines/des Verdrehsicherungselements mittels eines Federsteckers ermöglicht werden kann. Die Aufnahme 38 weist einen Zugang 38.2 in Form einer Ausfräsung auf. An diesem Zugang oder im Bereich dieses Zugangs kann ein Bolzen in die Durchgangsbohrung 38.1 am unteren Aufnahmeabschnitt 38a gesteckt werden, nämlich von oben in eine an der unteren Stirnfläche 32a.1 angeordneten Justageeinrichtung.

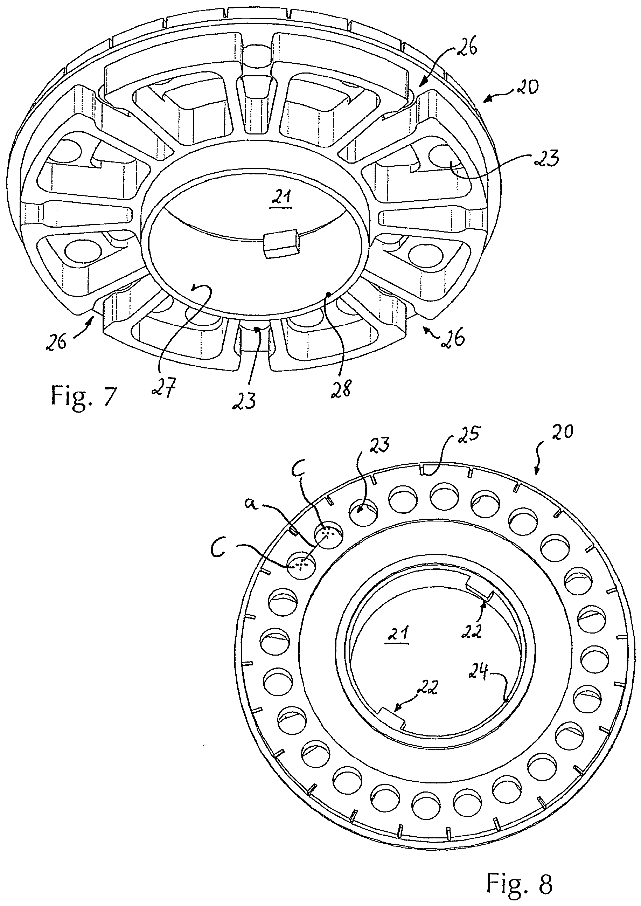

In der Figur 7 ist die Justageeinrichtung 20 von einer Unterseite gezeigt. Die Justageeinrichtung 20 kann als Flachring beschrieben werden. Die nicht dargestellte Spindel kann durch eine Durchführung 21 hindurchgeführt werden. An einer Innenmantelfläche 27 ist ein Verdrehanschlag 22 (wahlweise auch in der Funktion einer Zentrierung) angeordnet, insbesondere in Form einer Feder, welche geometrisch korrespondierend zu einer entsprechenden Nut der Spindel ausgebildet ist. Bevorzugt sind zwei Federn 22 vorgesehen, wie in Figur 8 gezeigt. Die Federn 22 sind an einem oberen Rand der Justageeinrichtung 20 angeordnet. An der Unterseite weist die Justageeinrichtung 20 einen hervorstehenden Rand 28 auf, welcher dank einer vergleichsweise großen Axialerstreckung eine exakte Ausrichtung oder Zentrierung gegenüber der Spindel erleichtert. An der Unterseite der Justageeinrichtung 20 sind Aussparungen 26 oder Radialschlitze vorgesehen, in welche ein Monteur mit einem Werkzeug (z.B. einem Schraubendreher) eingreifen kann, insbesondere von radial außen, um die Justageeinrichtung 20 und damit die Spindel zu verdrehen. Auf diese Weise kann die relative Verdrehposition justiert werden, insbesondere ohne Demontage irgendeiner Verkleidung oder irgendeines Gehäuses. Wie bereits in Bezug auf Fig. 1 beschrieben, weist die Justageeinrichtung eine Vielzahl von Kupplungspunkten 23 auf, welche geometrisch korrespondieren zu einem Mittel zur Verdrehsicherung, insbesondere einem Bolzen, ausgebildet sind. Die Kupplungspunkte 23 sind auf einem Teilkreis angeordnet, insbesondere konzentrisch um einen Mittelpunkt der Justageeinrichtung 20. Gemäß einer Variante sind die Kupplungspunkte 23 als Löcher ausgebildet und weisen zumindest annähernd denselben Durchmesser auf wie die in Figur 6 gezeigte Durchgangsbohrung 38.1.

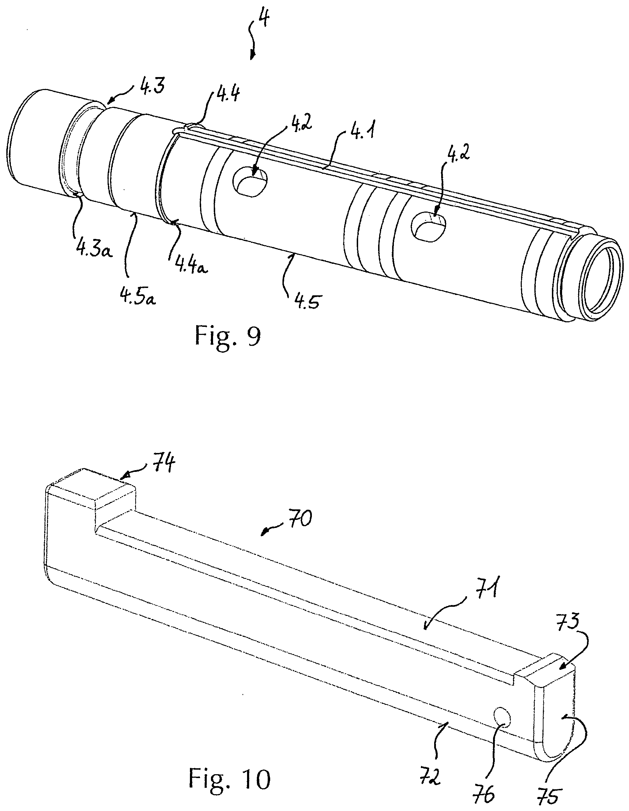

In der Figur 8 ist die Justageeinrichtung 20 in einer Draufsicht von oben gezeigt. Die Justageeinrichtung 20 weist eine ringförmige Auflagefläche 24 auf, welche zumindest teilweise auch durch die Federn 22 gebildet sein kann. Die Auflagefläche 24 ist geometrisch korrespondierend zu einem radial hervorstehenden Rand 4.4 (Figur 9) der Spindel ausgebildet. An einem Außenumfang der Justageeinrichtung 20 sind ferner Befestigungsmittel für eine Verkleidung oder ein Gehäuse vorgesehen. Die Befestigungsmittel sind in Form von Schlitzen 25 vorgesehen, welche sich in radialer Richtung erstrecken. Diese Ausgestaltung erleichtert ein Aufstecken von oben oder von der Seite in radialer Richtung. Wie gezeigt, ist die Justageeinrichtung 20 flach-ringförmig ausgebildet und weist in Bezug auf die Anordnung der Kupplungspunkte 23 bzw. Löcher eine wenigstens 12-zählige Rotationssymmetrie, im dargestellten Ausführungsbeispiel eine genau 24-zählige Rotationssymmetrie auf. D.h., dass nebeneinanderliegende Kupplungspunkte 23 bzw. Löcher bezogen auf ihr Zentrum bei der wenigstens 12-zähligen Rotationssymmetrie in einem Winkelabstand a von höchstens 30° zueinander liegen, und dass nebeneinanderliegende Kupplungspunkte bzw. Löcher bezogen auf ihr Zentrum bei der dargestellten 24-zähligen Rotationssymmetrie in einem Winkelabstand a von 15° zueinander liegen.

In der Figur 9 ist die Spindel 4 im Detail gezeigt. Die Spindel 4 weist zwei in Längsrichtung und gegenüberliegend zueinander angeordnete Axialnuten 4.1 auf. Die jeweilige Axialnut 4.1 erstreckt sich bis zu einem Absatz bzw. radial hervorstehenden Rand 4.4, sodass Federn in die Nuten 4.1 bis hin zum Rand 4.4 geschoben werden können. Die Spindel 4 weist ferner zwei in axialem Abstand zueinander eingebrachte Ausnehmungen 4.2 auf, welche eine Außenmantelfläche 4.5 durchstoßen. Durch die Ausnehmungen 4.2 kann jeweils ein Kabel, insbesondere Schleifringkabel hindurchgeführt werden. Oberhalb des Randes 4.4 ist ein Zentrierflächenabschnitt 4.5a vorgesehen, mittels welchem die Spindel 4 in der Kavität K zentriert werden kann. Durch den Rand 4.4 wird eine ringförmige Auflagefläche 4.4a definiert, an welcher die Justageeinrichtung zur Anlage kommen kann. Durch die umlaufende Nut 4.3 wird eine ringförmige Auflagefläche 4.3a definiert, über welche die Gewichtskraft einer Stativvorrichtung von der Spindel 4 auf die nicht dargestellte Axialsicherung 11 übertragen werden kann.

In der Figur 10 ist ein Sicherungselement 70 in Form eines Riegels im Detail gezeigt. Der Riegel 70 weist eine oberseitige Auflagefläche 71 auf, an welcher die in der Figur 9 gezeigte Auflagefläche 4.3a zur Anlage kommen kann. Ferner weist der Riegel 70 eine unterseitige, im vorliegenden Beispiel gekrümmte, Kontaktfläche 72 auf, mittels welcher der Riegel 70 in der Durchführung 36 an der Montageeinrichtung 30 lagerbar ist. Die Kontaktfläche 72 weist eine Krümmung mit vordefiniertem Krümmungsradius auf, wodurch eine geringe Kerbwirkung sichergestellt werden kann. Die gekrümmte Kontaktfläche 72 kann einen geringen Flächendruck sicherstellen. Der Riegel 70 weist eine U-förmige Querschnittsfläche 75 auf, welche entlang der gesamten Länge des Riegels 70 im Wesentlichen dieselbe Geometrie aufweist. Ein jeweiliges freies Ende des Riegels 70 weist einen Absatz 73, 74 auf, welcher eine Sicherung des Riegels 70 in der Durchführung 36 ermöglicht. Einer der Absätze ist kleiner als der andere.

Der Absatz 73 ist als Montagefase ausgebildet und kann ein Herausrutschen des Riegels 70 verhindern. Der Absatz 74 verhindert ebenfalls ein Herausrutschen. Der Absatz 74 ist bevorzugt derart hoch ausgebildet, dass der Riegel 70 nicht durch die korrespondierende Durchführung geschoben werden kann, sondern am Absatz 74 blockiert.

Im Bereich eines der beiden freien Enden weist der Riegel eine Durchgangsbohrung 76 auf, an welcher der Riegel 70 gesichert werden kann, insbesondere mittels eines Federsteckers.

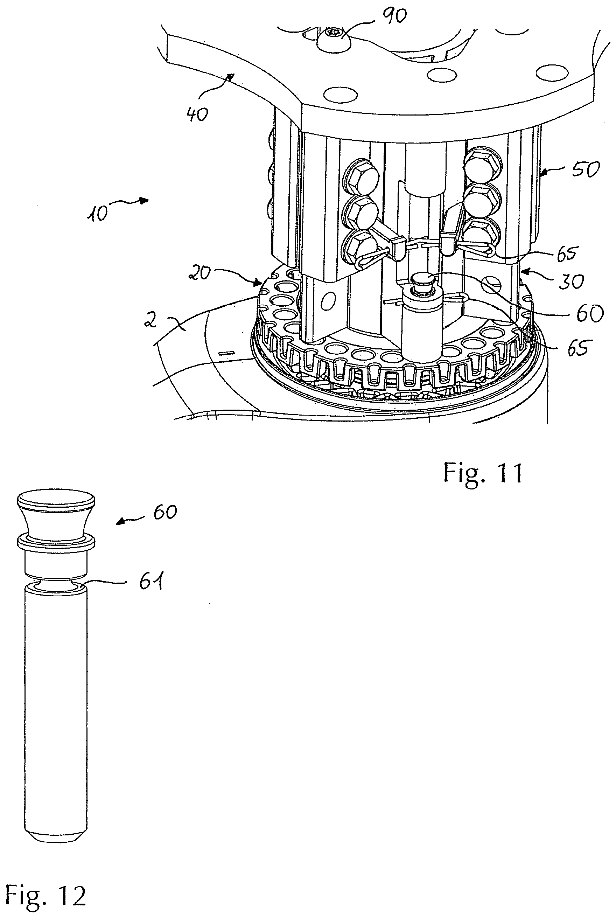

In der Figur 11 ist eine Montagevorrichtung 10 gezeigt, bei welcher ein Verdrehsicherungselement 60 mittels eines Federsteckers 65 an der Montageeinrichtung 30 in Eingriffsposition mit der Justageeinrichtung 20 gesichert ist. Wie aus Figur 12 hervorgeht, greift der Federstecker 65 in eine Nut 61 des Bolzens bzw. Verdrehsicherungselements 60 ein. Die beiden Riegel 70 sind ebenfalls mittels eines entsprechenden Federsteckers 65 gesichert, wobei die Federstecker jeweils in die entsprechende Durchgangsbohrung des jeweiligen Riegels 70 eingreifen.

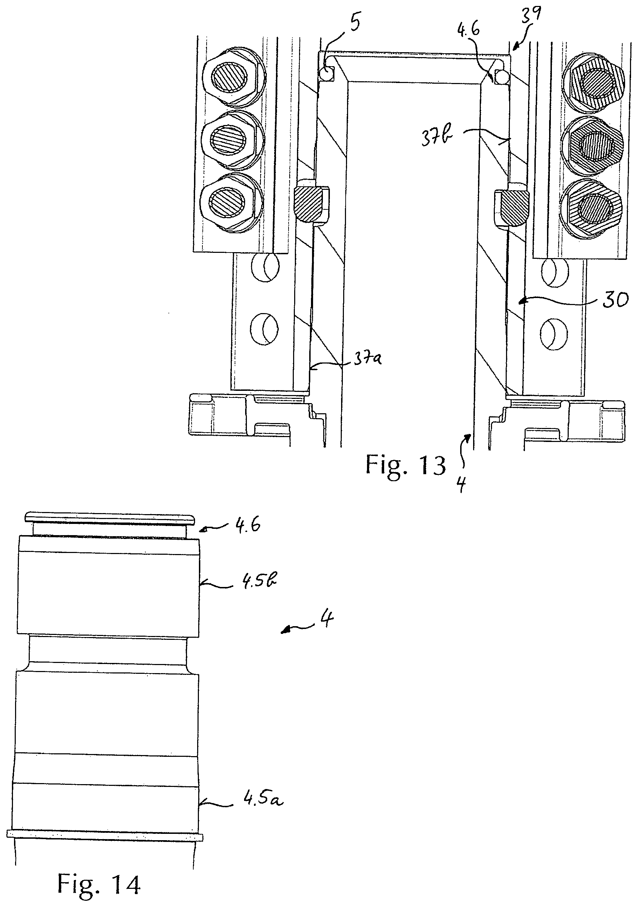

In den Figuren 13 und 14 ist gezeigt, auf welche Weise eine Montageerleichterung mittels zweier Passflächen 37a, 37b und korrespondierenden Flächenabschnitten 4.5a, 4.5b an der Spindel erfolgen kann. Die Spindel 4 weist einen Absatz 4.6 auf, an welchem ein O-Ring 5 angeordnet ist. An der Montageeinrichtung 30 ist dazu ein entsprechender Absatz 39 ausgebildet. Die zweite Passfläche 37b weist einen Innendurchmesser auf, welcher kleiner ist als derjenige der ersten Passfläche 37a. Auf diese Weise kann die Spindel 4 zum einen ohne das Risiko eines Verkantens montiert werden, zum anderen kann die Lagerung auf besonders stabile Weise an weit voneinander beabstandeten Flächenabschnitten 4.5a, 4.5b erfolgen.

### Bezugszeichenliste

- 1: Stativvorrichtung, insbesondere Deckenstativvorrichtung
- 2: (erster) Träger oder Tragarm
- 3: zweiter Träger oder Tragarm
- 4: Verbindungsbauteil, insbesondere Spindel
- 4.1: Verdrehsicherung, insbesondere Nut in Verbindungsbauteil, bevorzugt in Längsrichtung eingebrachte Axialnut
- 4.2: Ausnehmung
- 4.3: umlaufende Nut
- 4.3a: ringförmige Auflagefläche
- 4.4: Absatz bzw. radial hervorstehender Rand
- 4.4a: ringförmige Lagerfläche
- 4.5: Außenmantelfläche
- 4.5a: Zentrierflächenabschnitt bzw. erste Passfläche
- 4.5b: zweite Passfläche
- 4.6: Nut für O-Ring am freien Ende der Spindel
- 5: O-Ring

- 10: Montagevorrichtung
- 10a: anpassbarer Mechanismus, insbesondere einstellbare Drehkupplung
- 11: Axialsicherung

- 20: Justageeinrichtung, insbesondere Flachring
- 21: Durchführung
- 22: Verdrehanschlag, insbesondere Feder
- 23: Kupplungspunkt für jeweilige Verdrehposition, insbesondere Loch oder Bohrung
- 24: ringförmige Auflagefläche
- 25: Befestigungsmittel für Verkleidung, insbesondere Schlitz auf Oberseite
- 26: Aussparung, insbesondere an Unterseite, zugänglich von radial außen
- 27: Innenmantelfläche
- 28: Rand bzw. Zentrierung

- 30: Montageeinrichtung, insbesondere als Deckenrohr ausgebildeter Grundkörper, bevorzugt in Form eines Stranggussprofils
- 31: Außenmantelfläche
- 32: Stirnseite
- 32a: untere Stirnseite
- 32a.1: ebene untere Stirnfläche
- 32b: obere Stirnseite
- 32b.1: ebene obere Stirnfläche
- 33: Befestigungsabschnitt, insbesondere Steg
- 33.1: Radialflanke am Befestigungsabschnitt
- 33.1 a, 33.1b: gegenüberliegende, insbesondere parallel zueinander angeordnete Radialflanken
- 34: Befestigungsmittel, insbesondere Bohrung oder Gewindebohrung
- 36: Durchführung, insbesondere in tangentialer bzw. radialer Richtung
- 36.1: Auflagefläche
- 37: Innenmantelfläche, insbesondere zumindest abschnittsweise zylindrisch
- 37a: erste Passfläche
- 37b: zweite Passfläche
- 37.1: Fase, Kante oder Einfräsung
- 38: Aufnahme für Verdrehsicherungselement, insbesondere Sicherungsrohr
- 38a: unterer Aufnahmeabschnitt, insbesondere rohrartig
- 38.1: Durchgangsbohrung, insbesondere in axialer Richtung
- 38.2: Zugang, insbesondere Ausfräsung
- 38.3: oberseitige Zugangsfläche (Schnittstelle)
- 39: Absatz

- 40: Flanschplatte
- 41: Öffnung, insbesondere Bohrung
- 42a.1: ebene unterseitige Stirnfläche
- 43: Radialnut
- 50: Halter, insbesondere in Form eines Stranggussprofils
- 51: Befestigungsmittel, insbesondere Öffnung, bevorzugt Innengewindebohrung
- 52b.1: ebene oberseitige Stirnfläche
- 53: gegenüberliegende Backen
- 53a, 53b: einzelne Backenabschnitte einer Backe
- 54: Wellung
- 55: Radialkavität

- 60: Verdrehsicherungselement, insbesondere Bolzen
- 61: Nut

- 65: Federstecker

- 70: Sicherungselement zur Axialsicherung, insbesondere Riegel
- 71: oberseitige Auflagefläche
- 72: unterseitige Kontaktfläche
- 73: Absatz an einem freien Ende
- 74: Absatz an einem freien Ende
- 75: Querschnittsfläche
- 76: Durchgangsbohrung

- 80: Befestigungselement an Halter, insbesondere Schraube

- 90: Befestigungselement an Flanschplatte, insbesondere Schraube
- a: Winkelabstand
- C: Zentrum
- D: Drehachse
- G: Gewichtskraft
- K: Kavität
- x: radiale Richtung bzw. horizontale Richtung
- y: Querrichtung
- z: Längsrichtung bzw. axiale Richtung bzw. vertikale Richtung

## Patentansprüche

1. Montagevorrichtung (10) für eine Stativvorrichtung (1) zur Anordnung im Operationssaal und zum örtlichen Positionieren oder Verlagern einer medizintechnischen Einrichtung im Operationssaal, insbesondere durch eine Drehbewegung, umfassend
- eine sich in einer Längsrichtung entlang einer Drehachse (D) erstreckende Montageeinrichtung (30) mit einer in Längsrichtung ausgerichteten Kavität (K) zur Aufnahme eines gegenüber der Montageeinrichtung (30) um die Drehachse (D) drehbar lagerbaren Verbindungsbauteils (4) der Stativvorrichtung;
- eine Justageeinrichtung (20) zur verdrehfesten Anordnung des Verbindungsbauteils (4) in einer vordefinierten Verdrehposition relativ zur Montageeinrichtung (30), wobei die Justageeinrichtung (20) drehfest mit dem Verbindungsteil (4) verbindbar ausgebildet ist; und
- ein Verdrehsicherungselement (60);
**dadurch gekennzeichnet, dass** die Montagevorrichtung (10) eine um die Drehachse (D) einstellbare Drehkupplung (10a) zum Lagern des Verbindungsbauteils (4) an der Montageeinrichtung (30) bildet,
wobei die Justageeinrichtung (20) als ringförmige Geometrie oder ringförmige Scheibe ausgebildet ist, wobei die Justageeinrichtung (20) eine Mehrzahl von als Löcher oder Durchführungen ausgebildeten, auf einem Teilkreis ausgebildeten Kupplungspunkten (23) jeweils zum Festlegen einer von einer Mehrzahl von vordefinierten Verdrehpositionen der Drehkupplung aufweist, wobei die Montageeinrichtung (30) eine Aufnahme (38) für das Verdrehsicherungselement (60) aufweist, und
das Verdrehsicherungselement (60) dazu ausgebildet ist, zur Festlegung einer der vordefinierten Verdrehpositionen mit einem spezifischen Kupplungspunkt (23) der Mehrzahl von Kupplungspunkten (23) der Justageeinrichtung (20) zusammenzuwirken.

2. Montagevorrichtung (10) nach Anspruch 1, wobei die einstellbare Drehkupplung (10a) durch die Justageeinrichtung (20) und die Montageeinrichtung (30) gebildet ist, wobei die Justageeinrichtung (20) in einer vorbestimmten Verdrehposition um die Drehachse (D) relativ zur Montageeinrichtung (30) positionierbar und verdrehfest lagerbar ist.

3. Montagevorrichtung (10) nach Anspruch 1 oder 2, wobei die Justageeinrichtung (20) einen Verdrehanschlag (22) aufweist, insbesondere eine Nut oder Feder, welcher geometrisch korrespondierend zu einem am Verbindungsbauteil angeordneten Verdrehanschlag (4.1), insbesondere Nut oder Feder, ausgebildet ist.

4. Montagevorrichtung (10) nach einem der vorhergehenden Ansprüche, wobei die Aufnahme (38) eine rohrartige Aufnahme (38) für das Verdrehsicherungselement (60) ist, und/oder die Aufnahme (38) an einer Außenseite, insbesondere Außenmantelfläche (31) der Montageeinrichtung (30) angeordnet ist oder die Außenmantelfläche (31) zumindest abschnittsweise bildet.

5. Montagevorrichtung (10) nach Anspruch 4, wobei die Aufnahme (38) einen Zugang (38.2) aufweist, insbesondere einen Zugang von radial außen, wobei der Zugang (38.2) bevorzugt in Form einer Ausfräsung ausgebildet ist, und wobei der Zugang (38.2) bevorzugt eine oberseitige Zugangsfläche (38.3) aufweist, an welcher ein Verdrehsicherungselement (60) zur Anlage gebracht werden kann, insbesondere in einer vordefinierten Axialposition.

6. Montagevorrichtung (10) nach einem der vorhergehenden Ansprüche, wobei die Montagevorrichtung (10) eine Axialsicherung (11) aufweist, mittels welcher das Verbindungsbauteil in einer vordefinierten Axialposition an der Montageeinrichtung (30) lagerbar ist, insbesondere relativ verdrehbar zur Montageeinrichtung (30), wobei bevorzugt die Montageeinrichtung (30) zum Halten und verdrehbaren Lagern des Verbindungsbauteils in einer vorbestimmten axialen Relativposition ausgebildet ist, wobei bevorzugt an einer die Kavität (K) begrenzenden Innenmantelfläche (37) der Montageeinrichtung (30) eine Fase, Kante oder Einfräsung (37.1) vorgesehen ist.

7. Montagevorrichtung (10) nach Anspruch 6, wobei die Axialsicherung (11) eine tangential an der Montageeinrichtung (30) angeordnete Durchführung (36) umfasst, welche eine Außenmantelfläche (31) der Montageeinrichtung (30) durchstößt, bevorzugt an zwei Punkten, und welche eine/die Innenmantelfläche (37) der Montageeinrichtung (30) schneidet, wobei an der Durchführung (36) bevorzugt eine Auflagefläche (36.1) ausgebildet ist, welche eingerichtet ist, eine vom Verbindungsbauteil (4) ausgeübte Kraft auf die Montageeinrichtung (30) zu übertragen.

8. Montagevorrichtung (10) nach Anspruch 6 oder 7, wobei die Axialsicherung (11) mindestens ein Sicherungselement (70), insbesondere einen Riegel oder Bolzen umfasst, welches ausgelegt und eingerichtet ist, die Gewichtskraft der Stativvorrichtung (1) vom Verbindungsbauteil (4) auf die Montageeinrichtung (30) zu übertragen, wobei das Sicherungselement (70) bevorzugt tangential zur Montageeinrichtung (30) anordenbar ist und geometrisch korrespondierend zur Durchführung (36) ausgebildet ist, insbesondere an einer unterseitigen Kontaktfläche (72).

9. Montagevorrichtung (10) nach einem der vorhergehenden Ansprüche, wobei die Montagevorrichtung zwei Passflächen (37a, 37b) zum Ausbilden einer Lagerung des Verbindungsteils (4) relativ zur Montageeinheit (30) um die Drehachse (D) mit korrespondierend zu den Passflächen (37a, 37b) ausgebildeten Flächenabschnitten (4.5a, 4.5b) am Verbindungsteil (4) aufweist, wobei die zweite Passfläche (37b) einen Innendurchmesser aufweist, welcher kleiner ist als derjenige der ersten Passfläche (37a).

10. Montagevorrichtung (10) nach Anspruch 1, wobei die Aufnahme (38) an einer Außenmantelfläche (31) der Montageeinrichtung (30) angeordnet ist, wobei die Aufnahme (38) einen Zugang (38.2) von radial außen aufweist, wobei die Aufnahme (38) eine oberseitige Zugangsfläche (38.3) aufweist, an welcher ein Verdrehsicherungselement (60) zur Anlage bringbar ist, wobei die Justageeinrichtung (20) eine Nut oder Feder aufweist, welche geometrisch korrespondierend zu einem am Verbindungsbauteil (4) angeordneten Verdrehanschlag (4.1) ausgebildet ist, wobei die Montagevorrichtung (10) eine Axialsicherung (11) aufweist, welche eine an einer die Kavität (K) begrenzenden Innenmantelfläche (37) der Montageeinrichtung (30) vorgesehene Einfräsung (37.1) umfasst, wobei die Axialsicherung (11) eine tangential an der Montageeinrichtung (30) angeordnete Durchführung (36) umfasst, welche eine Außenmantelfläche (31) der Montageeinrichtung (30) durchstößt und die Innenmantelfläche (37) schneidet.

11. Montagevorrichtung (10) nach einem der vorhergehenden Ansprüche, wobei die Justageeinrichtung (20) flach-ringförmig ausgebildet ist und in Bezug auf die Anordnung der Kupplungspunkte (23) eine wenigstens 12-zählige Rotationssymmetrie, insbesondere eine wenigstens 24-zählige Rotationssymmetrie aufweist.

12. Montagevorrichtung (10) nach einem der Ansprüche 1 bis 11, wobei die Montageeinrichtung (30) eine von außen zugängliche Aufnahme (38) für ein Verdrehsicherungselement (60) zum Festlegen einer Verdrehposition aufweist, wobei die Montageeinrichtung (30) eingerichtet ist, mittels des Verdrehsicherungselements (60) ein Drehmoment zwischen dem Verbindungsbauteil (4) und einer Flanschplatte (40) zu übertragen.

13. Montagesystem mit einer Montagevorrichtung (10) nach einem der vorhergehenden Ansprüche, wobei das Montagesystem das Verbindungsbauteil (4) in Form einer Spindel aufweist, wobei an der Spindel (4) an ihrer Außenseite eine Axialnut (4.1) ausgebildet ist, welche geometrisch korrespondierend zu einem an der Justageeinrichtung (20) ausgebildeten Verdrehanschlag (22) ausgebildet ist.

14. Verwendung der Montagevorrichtung (10) nach Anspruch 12, an einer im Operationssaal angeordneten Stativvorrichtung (1) zum örtlichen Positionieren oder Verlagern einer medizintechnischen Einrichtung, insbesondere durch eine Drehbewegung, wobei die Montagevorrichtung (10) eine einstellbare Verdrehposition eines Verbindungsbauteils (4) der Stativvorrichtung (1) relativ zu einer Flanschplatte (40) festlegt, mittels welcher die Stativvorrichtung im Operationssaal befestigt ist.

## Claims

1. Mounting device (10) for a stand device (1) for arranging in the operating theatre and for locally positioning or displacing a medical-technical device in the operating theatre, in particular by means of a rotational movement, comprising
- a mounting unit (30), which extends in a longitudinal direction along an axis of rotation (D), and has a cavity (K) which is aligned in the longitudinal direction, for accommodating a - in view of the mounting unit (30) around the rotation axis (D) - rotatably supportable connecting member (4) of the stand device;
- an adjustment means (20) for rotatably fixedly arranging said connecting member (4) in a predefined position of rotation relative to said mounting unit (30), wherein the adjustment means (20) are configured to be connectable with connecting member (4) in a rotatably fixed manner; and
- a rotational lock element (60);
**characterized in that** the mounting device (10) forms a rotational coupling (10a) adjustable around the axis of rotation (D) for supporting the connecting member (4) on the mounting unit (30),
wherein the adjustment means (20) is formed of an annular geometry or is an annular disk, wherein the adjustment means (20) has a plurality of coupling points (23) formed as openings or passages and arranged on a pitch circle, each coupling point for defining one of a plurality of predefined positions of rotation of the rotational coupling, wherein the mounting unit (30) comprises a retainer (38) for the rotational lock element (60), and
the rotational lock element (60) is configured to cooperate with a specific coupling point (23) of the plurality of coupling points (23) of the adjustment means (20) to define one of the predefined positions of rotation.

2. Mounting device (10) according to claim 1, wherein the adjustable rotational coupling (10a) is formed by the adjustment means (20) and the mounting unit (30), wherein the adjustment means (20) can be positioned in a predefined position of rotation around the axis of rotation (D) relative to the mounting unit (30) and is supportable in a rotationally fixed manner.

3. Mounting device (10) according to claim 1 or 2, wherein the adjustment means (20) has a rotational stop (22), in particular a groove or a spring, which is geometrically designed to correspond to a rotational stop (4.1), in particular a groove or spring, arranged on the connecting member.

4. Mounting device (10) according to one of the preceding claims, wherein the retainer (38) is a retainer tubular retainer (38) for a rotational lock element (60), and/or the retainer (38) is arranged on an outer side, in particular an outer lateral surface (31) of the mounting unit (30), or forms the outer lateral surface (31) at least in sections.

5. Mounting device (10) according to claim 4, wherein the retainer (38) has an access (38.2), in particular an access from radially outside, wherein the access (38.2) is preferably in the form of a cut-out, and wherein the access (38.2) preferably has an upper access surface (38.3) on the upper side, against which a rotational lock element (60) can be brought into abutment, in particular in a predefined axial position.

6. Mounting device (10) according to one of the preceding claims, wherein the mounting device (10) has an axial lock (11), by means of which the connecting member can be supported on the mounting unit (30) in a predefined axial position, in particular rotatable relative to the mounting unit (30), wherein the mounting unit (30) is preferably designed to hold and rotatably support the connecting member in a predefined axial relative position, wherein preferably a chamfer, edge or milled recess (37.1) is provided on an inner lateral surface (37) of the mounting unit (30) surrounding cavity (K).

7. Mounting device (10) according to claim 6, wherein the axial lock (11) comprises a passage (36) arranged tangentially on the mounting unit (30), preferably at two points, and which passage passes through an outer lateral surface (31) of the mounting unit (30) and which intersects a/the inner lateral surface (37) of the mounting unit (30), wherein preferably the passage (36) forms a supporting surface (36.1), which is configured to transfer a force exerted by the connecting member (4) to the mounting unit (30).

8. Mounting device (10) according to claim 6 or 7, wherein the axial lock (11) comprises at least one securing element (70), in particular a latch or bolt, which is designed and configured to transfer the weight of the stand device (1) from the connecting member (4) to the mounting unit (30), wherein the securing element (70) can preferably be arranged tangentially to the mounting unit (30) and is designed geometrically corresponding to the passage (36), in particular on a lower side contact surface (72).

9. Mounting device (10) according to one of the preceding claims, wherein the mounting device has two mating faces (37a, 37b) for forming a support of the connecting member (4) relative to the mounting unit (30) around the axis of rotation (D) with surface portions (4.5a, 4.5b) corresponding to the mating faces (37a, 37b) on the connecting member (4), wherein the second mating face (37b) has an inner diameter, which is smaller than the one of the first mating face (37a).

10. Mounting device (10) according to claim 1, wherein the retainer (38) is disposed on an outer lateral surface (31) of the mounting unit (30), said retainer (38) having an access (38.2) from radially outside, wherein the retainer (38) has an upper access surface (38.3), against which the rotational lock element (60) can abut, wherein the adjustment means (20) has a groove or spring which corresponds geometrically to a rotational stop (4.1) arranged on the connecting member (4), wherein the mounting device (10) has an axial lock (11) which comprises a milled recess (37.1) provided on an inner lateral surface (37) of the mounting unit (30) surrounding the cavity (K), wherein the axial lock (11) comprises a passage (36) arranged tangentially on the mounting unit (30), which passes through an outer lateral surface (31) of the mounting unit (30) and intersects the inner lateral surface (37).

11. Mounting device (10) according to one of the preceding claims, wherein the adjustment means (20) is of flat ring design and, with respect to the arrangement of the coupling points (23), has an at least 12-fold rotational symmetry, in particular an at least 24-fold rotational symmetry.

12. A mounting device (10) according to one of the claims 1 to 11, wherein the mounting unit (30) has a retainer (38) being accessible from outside for a rotational lock element (60) for defining a position of rotation, wherein the mounting unit (30) is configured to transfer a torque between the connecting member (4) and a flange plate (40) by means of the rotational lock element (60).

13. Mounting system having a mounting device (10) according to one of the preceding claims, wherein the mounting system has the connecting member (4) in the form of a spindle, wherein an axial groove (4.1) is formed on the spindle on its outer surface, which is formed geometrically corresponding to a rotational stop (22) formed on the adjustment means (20).

14. Use of a mounting device (10) according to claim 12 on a stand device (1) arranged in the operating theatre for local positioning or displacing a medical-technical device, in particular by a rotational movement, wherein the mounting device (10) defines an adjustable position of rotation of a connecting member (4) of the stand device (1) relative to a flange plate (40) by means of which the stand device is mounted in the operating theatre.

## Revendications

1. Dispositif de montage (10) pour un dispositif de support (1) destiné à être disposé dans la salle d'opération et pour le positionnement ou le déplacement local d'un dispositif médico-technique dans la salle d'opération, en particulier par un mouvement rotatif, comprenant
- un moyen de montage (30) qui s'étend dans une direction longitudinale le long d'un axe de rotation (D) et présente une cavité (K) alignée dans la direction longitudinale pour recevoir un élément de liaison (4) du dispositif de support pouvant être monté rotatif autour de l'axe de rotation (D) par rapport au moyen de montage (30) ;
- un moyen de réglage (20) pour disposer l'élément de liaison (4) de manière fixe en rotation dans une position de rotation prédéfinie par rapport au moyen de montage (30), dans lequel le moyen de réglage (20) est conçu de façon à pouvoir être relié à l'élément de liaison (4) de manière fixe en rotation ; et
- un élément anti-rotation (60) ;
**caractérisé en ce que** le dispositif de montage (10) forme un accouplement rotatif (10a) réglable autour de l'axe de rotation (D) pour supporter l'élément de liaison (4) sur le moyen de montage (30),
dans lequel le moyen de réglage (20) est de conception géométrique annulaire ou se présente sous la forme d'un disque annulaire, dans lequel le moyen de réglage (20) présente plusieurs points d'accouplement (23), sous la forme de trous ou de douilles, prévus sur un cercle partiel, destinés à fixer chacun une position de rotation parmi plusieurs positions de rotation prédéfinies de l'accouplement rotatif, dans lequel le moyen de montage (30) comporte un logement (38) pour l'élément anti-rotation (60), et l'élément anti-rotation (60) est conçu pour coopérer avec un point d'accouplement (23) spécifique des plusieurs points d'accouplement (23) du moyen de réglage (20) pour la fixation de l'une des positions de rotation prédéfinies.

2. Dispositif de montage (10) selon la revendication 1, dans lequel l'accouplement rotatif (10a) réglable est formé par le moyen de réglage (20) et le moyen de montage (30), dans lequel le moyen de réglage (20) peut être positionné et monté de manière fixe en rotation dans une position de rotation prédéterminée autour de l'axe de rotation (D) par rapport au moyen de montage (30).

3. Dispositif de montage (10) selon la revendication 1 ou la revendication 2, dans lequel le moyen de réglage (20) présente une butée anti-rotation (22), en particulier une languette ou rainure, qui est conçue pour correspondre géométriquement à une butée anti-rotation (4.1), en particulier une languette ou rainure, disposée sur l'élément de liaison.

4. Dispositif de montage (10) selon l'une des revendications précédentes, dans lequel le logement (38) est un logement (38) tubulaire pour l'élément anti-rotation (60), et/ou le logement (38) est disposé sur un côté extérieur, en particulier une surface périphérique extérieure (31) du moyen de montage (30), ou forme au moins par segments la surface périphérique extérieure (31).

5. Dispositif de montage (10) selon la revendication 4, dans lequel le logement (38) présente un accès (38.2), en particulier un accès depuis l'extérieur radialement, dans lequel l'accès (38.2) est de préférence conçu sous la forme d'un évidement fraisé, et dans lequel l'accès (38.2) présente de préférence une surface d'accès supérieure (38.3), contre laquelle un élément anti-rotation (60) peut être amené en appui, en particulier dans une position axiale prédéfinie.

6. Dispositif de montage (10) selon l'une des revendications précédentes, le dispositif de montage (10) présentant un moyen de fixation axial (11) au moyen duquel l'élément de liaison peut être monté sur le moyen de montage (30) dans une position axiale prédéfinie, en particulier de manière à pouvoir tourner relativement par rapport au moyen de montage (30), le moyen de montage (30) étant de préférence conçu pour maintenir et supporter, de manière à ce qu'il puisse tourner, l'élément de liaison dans une position axiale relative prédéterminée, un chanfrein, un bord ou un fraisage (37.1) étant de préférence prévu sur une surface périphérique intérieure (37) du moyen de montage (30) qui délimite la cavité (K).

7. Dispositif de montage (10) selon la revendication 6, dans lequel le moyen de fixation axial (11) comprend une douille (36) disposée de manière tangentielle sur le moyen de montage (30), laquelle douille transperce une surface périphérique extérieure (31) du moyen de montage (30), de préférence en deux points, et laquelle douille coupe une/la surface périphérique intérieure (37) du moyen de montage (30), dans lequel une surface d'appui (36.1) est de préférence constituée sur la douille (36), laquelle surface d'appui est agencée pour transférer une force exercée par l'élément de liaison (4) au moyen de montage (30).

8. Dispositif de montage (10) selon la revendication 6 ou la revendication 7, dans lequel le moyen de fixation axial (11) comprend au moins un élément de fixation (70), en particulier un verrou ou un boulon, lequel est configuré et agencé pour transférer la force de poids du dispositif de support (1) de l'élément de liaison (4) au moyen de montage (30), dans lequel l'élément de fixation (70) peut de préférence être disposé de manière tangentielle sur le moyen de montage (30) et est conçu pour correspondre géométriquement à la douille (36), en particulier sur une surface de contact inférieure (72).

9. Dispositif de montage (10) selon l'une des revendications précédentes, le dispositif de montage comportant deux surfaces d'ajustage (37a, 37b) pour la constitution d'un montage de l'élément de liaison (4) relativement par rapport à l'unité de montage (30) autour de l'axe de rotation (D) avec des segments de surface (4.5a, 4.5b) conçus pour correspondre aux surfaces d'ajustage (37a, 37b) sur l'élément de liaison (4), dans lequel la deuxième surface d'ajustage (37b) présente un diamètre intérieur qui est plus petit que celui de la première surface d'ajustage (37a).

10. Dispositif de montage (10) selon la revendication 1, dans lequel le logement (38) est disposé sur une surface périphérique extérieure (31) du moyen de montage (30), dans lequel le logement (38) présente un accès (38.2) depuis l'extérieur radialement, dans lequel le logement (38) présente une surface d'accès supérieure (38.3), contre laquelle un élément anti-rotation (60) peut être amené en appui, dans lequel le moyen de réglage (20) présente une rainure ou languette qui est conçue pour correspondre géométriquement à une butée anti-rotation (4.1) disposée sur l'élément de liaison (4), le dispositif de montage (10) comportant un moyen de fixation axial (11) qui comprend un fraisage (37.1) prévu sur une surface périphérique intérieure (37) du moyen de montage (30) qui délimite la cavité (K), le moyen de fixation axial (11) comprenant une douille (36) disposée de manière tangentielle sur le moyen de montage (30), laquelle douille transperce une surface périphérique extérieure (31) du moyen de montage (30) et coupe la surface périphérique intérieure (37).

11. Dispositif de montage (10) selon l'une des revendications précédentes, dans lequel le moyen de réglage (20) est de conception annulaire plate et présente, par rapport à la disposition des points d'accouplement (23), une symétrie de rotation d'au moins 12 fois, en particulier une symétrie de rotation d'au moins 24 fois.

12. Dispositif de montage (10) selon l'une des revendications 1 à 11, dans lequel le moyen de montage (30) comporte un logement (38) accessible depuis l'extérieur pour un élément anti-rotation (60) pour la fixation d'une position de rotation, dans lequel le moyen de montage (30) est configuré pour transmettre un couple de rotation entre l'élément de liaison (4) et une plaque de bride (40) au moyen de l'élément anti-rotation (60).

13. Système de montage avec un dispositif de montage (10) selon l'une des revendications précédentes, le système de montage comportant l'élément de liaison (4) sous la forme d'une broche, une languette axiale (4.1) étant constituée sur la broche (4) sur son côté extérieur, laquelle languette axiale est conçue pour correspondre géométriquement à une butée anti-rotation (22) constituée sur le moyen de réglage (20).

14. Utilisation du dispositif de montage (10) selon la revendication 12, sur un dispositif de support (1) disposé dans la salle d'opération pour le positionnement ou le déplacement local d'un dispositif médico-technique, en particulier par un mouvement rotatif, dans laquelle le dispositif de montage (10) fixe une position de rotation réglable d'un élément de liaison (4) du dispositif de support (1) relativement par rapport à une plaque de bride (40), au moyen de laquelle le dispositif de support est fixé dans la salle d'opération.
